(19) Europäisches Patentamt
European Patent Office
Office européen des brevets

(11) Veröffentlichungsnummer: **0 165 209**
**B1**

# (12) EUROPÄISCHE PATENTSCHRIFT

(45) Veröffentlichungstag der Patentschrift: **19.07.89**

(51) Int. Cl.[4]: **C 07 C149/273**, C 09 K 15/14

(21) Anmeldenummer: **85810259.3**

(22) Anmeldetag: **06.06.85**

## (54) o,p-Bifunktionalisierte, o'-substituierte Phenole.

(30) Priorität: **12.06.84 CH 2835/84**

(43) Veröffentlichungstag der Anmeldung: **18.12.85 Patentblatt 85/51**

(45) Bekanntmachung des Hinweises auf die Patenterteilung: **19.07.89 Patentblatt 89/29**

(84) Benannte Vertragsstaaten: **BE DE FR GB IT NL**

(56) Entgegenhaltungen:
**DE–A– 1 907 565**
**DE–A– 2 838 273**
**US–A– 3 903 173**
**CHEMICAL ABSTRACTS SERVICE REGISTRY HANDBOOK, Number section 1965-1971, Registry Numbers 12700-00-4 through 17799-99-4, Seite 5148R, American Chemical Society, US**
**Die Akte enthält technische Angaben, die nach dem Eingang der Anmeldung eingereicht wurden und die nicht in dieser Patentschrift enthalten sind.**

(73) Patentinhaber: **CIBA-GEIGY AG**
**Klybeckstrasse 141**
**CH-4002 Basel (CH)**

(72) Erfinder: **Meier, Hans Rudolf, Dr.**
**Ch. des Epinettes 12**
**CH-1723 Marly (CH)**

**EP 0 165 209 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue bifunktionalisierte Phenole, Zusammensetzungen enthaltend diese Verbindungen sowie ein Verfahren zum Stabilisieren von organischem Material unter Verwendung dieser Stoffe.

Bifunktionalisierte thiomethylhaltige Phenole sind bekannt. In der US-A-4 091 037 wird ein Verfahren zur Herstellung von Alkylthiomethylphenolen beschrieben, wobei 2,4-Bis(ethylthiomethyl)phenol spezifisch erwähnt ist.

In der US-A-3 660 352 wird der Einsatz von 2,4,6-Trialkyl-bis(3,5-alkylthiomethyl)-phenolen als Antioxidantien in Polymeren vorgeschlagen. Schliesslich beschreibt die US-A-3 227 677 unter anderem 2,6-Bis(alkoxycarbonylalkylenthiomethyl)-4-alkylphenole als Stabilisatoren für Polyolefine.

Nach der GB-A-1 184 533 sind 2,4-Bis(alkyltiomethyl)-3,6-dialkylphenole als Stabilisatoren für organische Polymere sowie für synthetische Oele einsetzbar.

Die DE-A-28 38 273 beschreibt ein neues Verfahren zur Herstellung von Alkyl- bzw. Arylthiomethylphenolen, die als Zwischenprodukte zur Synthese von Pflanzenschutzmitteln der in der DE-B-1 910 588 und DE-A-2 122 311 beschriebenen Art dienen, indem man bestimmte Hydroxymethylphenole bei Temperaturen zwischen 20 und 200 °C mit Mercaptanen oder Thiophenolen umsetzt.

Die Erfindung betrifft Verbindungen der Formel I

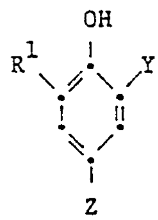

(I)

worin $R^1$ $C_2$-$C_{20}$-Alkyl, $C_3$-$C_{20}$-Alkenylmethyl, $C_3$-$C_{20}$-Alkinylmethyl, $C_5$-$C_{12}$-Cycloalkyl, Phenyl, 1- oder 2-Naphthyl, $C_7$-$C_{14}$-Alkaryl oder $C_7$-$C_{14}$-Aralkyl bedeutet, Y $—CH_2—S—R^2$ ist, Z $—CH_2—S—R^3$ bedeutet oder worin Y und/oder Z ein Rest

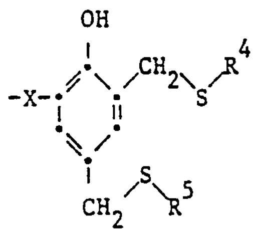

ist, worin $R^2$, $R^3$, $R^4$ und $R^5$ unabhängig voneinander $C_1$-$C_{20}$-Alkyl, durch einen Phenylrest und/oder eine oder zwei Hydroxygruppen substituiertes $C_2$-$C_{20}$-Alkyl, $C_3$-$C_{20}$-Alkenylmethyl, $C_3$-$C_{20}$-Alkinylmethyl, $C_5$-$C_{12}$-Cycloalkyl, Phenyl, 1- oder 2-Naphthyl, $C_7$-$C_{14}$-Alkaryl, $C_7$-$C_{14}$-Aralkyl oder durch Hydroxy in 2-Position substituiertes $C_5$-$C_7$-Cycloalkyl oder 1,3-Benzothiazol-2-yl sind, oder Reste der Formeln $—C(R^6R^7)—(CHR^8)_m—W$, $—(CH_2)_2—OCO—R^{15}$ oder

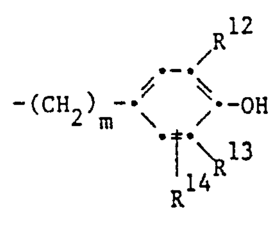

bedeuten, worin m 0, 1 oder 2 ist, $R^6$, $R^7$ und $R^8$ unabhängig voneinander Wasserstoff oder $C_1$-$C_6$-Alkyl sind, W ein Rest $—COR^9$, $—CN$, $—COOR^{10}$ oder $—CON(R^{10}R^{11})$ ist, wobei $R^9$ Wasserstoff, $C_1$-$C_{20}$-Alkyl, Phenyl, 1- oder 2-Naphthyl $C_5$-$C_{12}$-Cycloalkyl, $C_7$-$C_{14}$-Aralkyl oder $C_7$-$C_{14}$-Alkaryl ist und worin $R^{10}$ und $R^{11}$ unabhängig voneinander die Bedeutungen von $R^9$ besitzen, oder zusätzlich durch eine Hydroxy- oder Cyanogruppe substituiertes $C_2$-$C_{20}$-Alkyl sind oder durch ein bis fünf $—O—$, $—S—$, $—N(CH_3)—$ oder $—N(C_2H_5)—$ unterbrochenes gegebenenfalls mit einer Hydroxygruppe substituiertes $C_3$-$C_{20}$-Alkyl bedeuten, wobei gegebenenfalls mehrfach auftretende Heteroatome durch mindestens eine Methylengruppe getrennt sein müssen, worin $R^{10}$ oder $R^{11}$ $C_2$-$C_{12}$-Alkenyl, $C_2$-$C_{12}$-Alkinyl, durch ein oder zwei $—NO_2$, $—Cl$, $—Br$, $—OCH_3$ oder $—COOR^{18}$ substituiertes Phenyl oder eine Gruppe

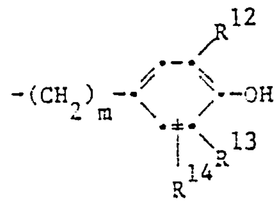

bedeuten, oder worin $R^{10}$ und $R^{11}$ zusammen mit dem Stickstoffatom einen fünf-, sechs- oder siebengliedrigen heterozyklischen Ring bilden, der gegebenenfalls noch ein weiteres Heteroatom enthalten kann, worin $R^{12}$, $R^{13}$ und $R^{14}$ unabhängig voneinander Wasserstoff, $C_1$-$C_{20}$-Alkyl, Cyclohexyl oder Phenyl sind, worin $R^{15}$ $C_1$-$C_{20}$-Alkyl, $C_5$-$C_{12}$-Cycloalkyl, $C_7$-$C_{14}$-Aralkyl, $C_7$-$C_{14}$-Alkaryl, Phenyl, 1- oder 2-Naphthyl oder ein Rest

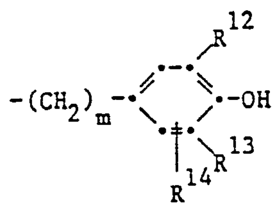

bedeutet, worin X —$C(R^{16}R^{17})$—, —S— oder —S—S— ist, wobei $R^{16}$ und $R^{17}$ unabhängig voneinander Wasserstoff, $C_1$-$C_6$-Alkyl, Cyclohexyl oder Phenyl bedeuten, und worin schliesslich $R^{18}$ $C_1$-$C_6$-Alkyl, Cyclohexyl, Phenyl, Benzyl oder Tolyl ist.

Bevorzugt werden Verbindungen der Formel I, worin Y —$CH_2$—S—$R^2$ und Z —$CH_2$—S—$R^3$ bedeuten.

Ebenfalls von Interesse sind Verbindungen der Formel I, worin Y —$CH_2$—S—$R^2$ und Z —$CH_2$—S—$R^3$ bedeuten, worin $R^1$ verzweigtes $C_3$-$C_{12}$-Alkyl, $C_5$-$C_9$-Cycloalkyl, Phenyl oder $C_7$-$C_9$-Aralkyl ist, worin $R^2$ und $R^3$ unabhängig voneinander $C_4$-$C_{14}$-Alkyl, durch ein Phenyl und/oder eine oder zwei Hydroxygruppen substituiertes $C_2$-$C_{14}$-Alkyl, Allyl, Propargyl, $C_5$-$C_9$-Cycloalkyl, 2-Hydroxycyclohexyl, Phenyl, 1,3-Benzothiazol-2-yl oder $C_7$-$C_9$-Aralkyl sind, oder ein Rest —$C(R^6R^7)$—$(CHR^8)_m$—W, —$(CH_2)_2$—OCO—$R^{15}$ oder

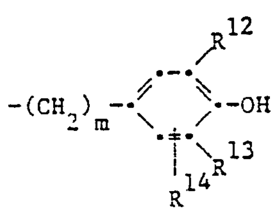

sind, worin m 0, 1 oder 2 bedeutet, und worin $R^6$, $R^7$ und $R^8$ unabhängig voneinander Wasserstoff oder Methyl sind, W —$COOR^{10}$ oder —$CON(R^{10}R^{11})$ bedeutet, wobei $R^{10}$ und $R^{11}$ unabhängig voneinander Wasserstoff, $C_1$-$C_{12}$-Alkyl, Phenyl, $C_5$-$C_9$-Cycloalkyl, $C_7$-$C_9$-Aralkyl, 2-Hydroxyethyl, 2-Cyanethyl oder durch ein bis drei —O— unterbrochenes gegebenenfalls durch eine Hydroxygruppe substituiertes $C_3$-$C_{12}$-Alkyl sind, wobei gegebenenfalls mehrfach auftretende Sauerstoffatome durch mindestens eine Methylengruppe getrennt sein müssen, worin $R^{10}$ und $R^{11}$ Allyl, Propargyl, durch ein —Cl, —$COOCH_3$ oder —$OCH_3$ substituiertes Phenyl oder einen Rest

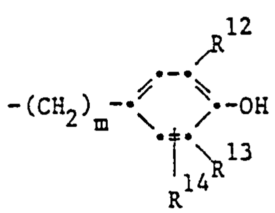

bedeuten, oder worin $R^{10}$ und $R^{11}$ zusammen mit dem gemeinsamen Stickstoffatom einen Pyrrol-, Piperidin-, Pyrrolidin-, Hexamethylenimin- oder Morpholinring bilden, worin $R^{12}$, $R^{13}$ und $R^{14}$ unabhängig voneinander Wasserstoff oder $C_1$-$C_{12}$-Alkyl sind, worin $R^{15}$ $C_1$-$C_{12}$-Alkyl, $C_5$-$C_9$-Cycloalkyl, $C_7$-$C_9$-Aralkyl, Phenyl oder ein Rest

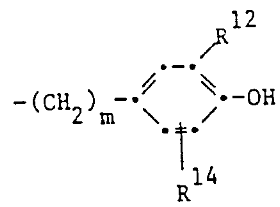

ist.

Insbesondere werden bevorzugt Verbindungen der Formel I, worin Y $—CH_2—S—R^2$ und Z $—CH_2—S—R^3$ sind, worin $R^1$ tert.-Butyl, Cyclohexyl, Phenyl oder Benzyl ist, worin $R^2$ und $R^3$ unabhängig voneinander $C_4$-$C_{14}$-Alkyl, 2-Hydroxyethyl, 2,3-Dihydroxypropyl, 1-Phenyl-2-hydroxyethyl, Cyclohexyl, Phenyl, Benzyl oder ein Rest $—(CH_2)_{m+1}—W$ bedeuten, wobei W $—COOR^{10}$ oder $—CON(R^{10}R^{11})$ ist, m 0, 1 oder 2 bedeutet, und worin schliesslich $R^{10}$ und $R^{11}$ unabhängig voneinander Wasserstoff, $C_1$-$C_{12}$-Alkyl, Phenyl, Cyclohexyl, Benzyl, 2-Hydroxyethyl, 2-Hydroxypropyl, 3-Oxabutyl oder $—(CH_2\text{-}CH_2—O\text{-})_n—CH_2—CH_2—OH$ bedeuten, wobei n 1, 2 oder 3 ist, oder wobei $R^{10}$ und $R^{11}$

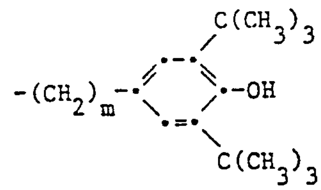

sind oder zusammen mit dem gemeinsamen Stickstoffatom einen Piperidin- oder Morpholinring bilden.

Auch werden Verbindungen der Formel I bevorzugt, worin Y $—CH_2—S—R^2$ und Z $—CH_2—S—R^3$ sind, $R^1$ tert.-Butyl, Cyclohexyl, Phenyl oder Benzyl bedeutet, und worin $R^2$ und $R^3$ unabhängig voneinander $C_4$-$C_{14}$-Alkyl, 2-Hydroxyethyl, 2,3-Dihydroxypropyl, 1-Phenyl-2-hydroxyethyl, Cyclohexyl, Phenyl oder Benzyl darstellen.

Ebenfalls bevorzugt werden Verbindungen der Formel I, worin Y $—CH_2—S—R^2$ und Z $—CH_2—S—R^3$ sind, $R^1$ tert.-Butyl, Cyclohexyl, Phenyl oder Benzyl bedeutet, worin $R^2$ und $R^3$ Reste der Formel $—(CH_2)_{m+1}—W$ sind, wobei W $—COOR^{10}$ oder $—CON(R^{10}R^{11})$ bedeutet und wobei $R^{10}$ und $R^{11}$ unabhängig voneinander Wasserstoff, $C_1$-$C_{12}$-Alkyl, Phenyl, Cyclohexyl, Benzyl, 2-Hydroxyethyl, 2-Hydroxypropyl, 5-Hydroxy-3-oxapentyl oder 3-Oxabutyl bedeuten, oder ein Rest

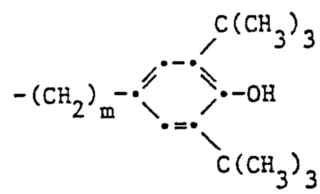

sind oder wobei $R^{10}$ und $R^{11}$ zusammen mit dem gemeinsamen Stickstoffatom einen Piperidin- oder Morpholinring bilden, und worin schliesslich m 0, 1 oder 2 ist.

Ganz besonders bevorzugt werden Verbindungen der Formel I, worin Y $—CH_2—S—R^2$ ist und worin Z $—CH_2—S—R^3$ bedeutet, worin $R^1$ tert.-Butyl, Phenyl, Cyclohexyl oder Benzyl bedeutet, worin $R^2$ und $R^3$ Reste der Formel

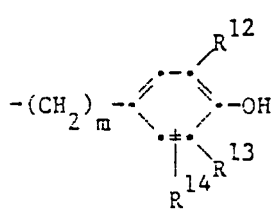

sind, wobei m 0, 1 oder 2 bedeutet und worin schliesslich $R^{12}$, $R^{13}$ und $R^{14}$ unabhängig voneinander Wasserstoff oder $C_1$-$C_{12}$-Alkyl sind.

Besonders bevorzugt werden Verbindungen der Formel I, worin Y $—CH_2—S—R^2$ ist und Z $—CH_2—S—R^3$ bedeutet, und worin $R^1$ tert.-Butyl ist und $R^2$ und $R^3$ unabhängig voneinander $C_4$-$C_{14}$-Alkyl sind.

Ebenfalls von Interesse sind Verbindungen der Formel I, worin X $—CH_2—$, $—CH(CH_3)—$, $—C(CH_3)_2—$ oder $—S—$ bedeutet.

$R^1$ als $C_2$-$C_{20}$-Alkyl bedeutet beispielsweise Ethyl, n-Propyl, Isopropyl, n-Butyl, sek.-Butyl, tert.-Butyl, n-Pentyl, Isopentyl, n-Hexyl, 1,1-Dimethylbutyl, n-Heptyl, n-Octyl, 2-Ethylhexyl, n-Nonyl, n-Decyl, 1,1,3,3-Tetramethylbutyl, 1,1,3,3-Tetramethylhexyl, n-Undecyl, n-Dodecyl, 1,1,3,3,5,5-Hexamethylhexyl, n-Tetradecyl, n-Hexadecyl, n-Octadecyl oder n-Eicosyl.

Besonders bevorzugt wird $R^1$ als $C_4$-$C_{20}$-Alkyl und bedeutet dann beispielsweise n-Butyl, sek.-Butyl, tert.-Butyl, n-Pentyl, Isopentyl, n-Hexyl, 1,1-Dimethylbutyl, n-Octyl, 2-Ethylhexyl, n-Decyl, 1,1,3,3-Tetramethylbutyl, n-Dodecyl, 1,1,3,3,5,5-Hexamethylhexyl, n-Tetradecyl, n-Hexadecyl, n-Octadecyl oder n-Eicosyl.

Insbesondere bevorzugt wird $R^1$ als verzweigtes $C_3$-$C_{12}$-Alkyl und ist dann beispielsweise Isopropyl, sek.-Butyl, tert.-Butyl, Isopentyl, 2-Ethylhexyl, 1,1-Dimethylbutyl, 1,1,3,3-Tetramethylbutyl, 1,1,3,3-Tetramethylhexyl oder 1,1,3,3,5,5-Hexamethylhexyl. Von besonderem Interesse ist $R^1$ als tert.-Butyl.

$R^2$, $R^3$, $R^4$, $R^5$, $R^9$, $R^{10}$, $R^{11}$, $R^{12}$, $R^{13}$, $R^{14}$ oder $R^{15}$ als $C_1$-$C_{20}$-Alkyl bedeuten zusätzlich zu den für $R^1$ beispielhaft erwähnten Resten noch Methyl.

$R^2$, $R^3$, $R^4$ und $R^5$ sind vorzugsweise $C_4$-$C_{14}$-Alkyl und bedeuten dann beispielsweise n-Butyl, sek.-Butyl, tert.-Butyl, n-Pentyl, Isopentyl, n-Hexyl, n-Heptyl, n-Octyl, 2-Ethylhexyl, 1,1,3,3-Tetramethylbutyl (im weiteren t-Octyl genannt), n-Nonyl, n-Decyl, n-Dodecyl, 1,1,3,3,5,5-Hexamethylhexyl und 2,2,4,6,6-Pentamethylhept-4-yl (im weiteren t-Dodecyl genannt) oder n-Tetradecyl.

Ganz besonders bevorzugt sind $R^2$, $R^3$, $R^4$ und $R^5$ als $C_8$-$C_{14}$-Alkyl und insbesondere als n-Octyl, 2-Ethylhexyl, t-Octyl, n-Dodecyl oder als t-Dodecyl.

$R^9$, $R^{10}$, $R^{11}$, $R^{12}$, $R^{13}$, $R^{14}$ und $R^{15}$ bedeuten vorzugsweise $C_1$-$C_{12}$-Alkyl und sind dann beispielsweise Methyl, Ethyl, n-Propyl, Isopropyl, n-Butyl, sek.-Butyl, tert.-Butyl, n-Pentyl, Isopentyl, n-Hexyl, n-Heptyl, n-Octyl, 2-Ethylhexyl, n-Nonyl, n-Decyl oder n-Dodecyl.

$R^6$, $R^7$, $R^8$, $R^{16}$, $R^{17}$ und $R^{18}$ als $C_1$-$C_6$-Alkyl bedeuten beispielsweise Methyl, Ethyl, n-Propyl, Isopropyl, n-Butyl, sek.-Butyl, tert.-Butyl, n-Pentyl, Isopentyl oder n-Hexyl.

$R^6$, $R^7$, $R^8$, $R^{16}$, $R^{17}$ und $R^{18}$ sind bevorzugt geradkettige $C_1$-$C_6$-Alkylreste und besonders bevorzugt Methyl.

$R^1$, $R^2$, $R^3$, $R^4$ und $R^5$ als $C_3$-$C_{20}$-Alkenylmethyl sind beispielsweise Allyl, Isopropenyl, Oct-7-enyl, Dodec-11-enyl, Oleyl oder Octadec-17-enyl. Bevorzugt wird Allyl.

$R^{10}$ und $R^{11}$ als $C_2$-$C_{12}$-Alkenyl bedeuten beispielsweise Vinyl, Allyl, But-3-enyl, Pent-4-enyl, Hex-5-enyl, Oct-7-enyl, Dec-9-enyl oder Dodec-11-enyl. Bevorzugt werden Vinyl oder Allyl. Bedeuten $R^1$, $R^2$, $R^3$, $R^4$ oder $R^5$ $C_3$-$C_{20}$-Alkinylmethyl, so handelt es sich beispielsweise um Propargyl, But-3-inyl, Hex-5-inyl, Oct-7-inyl, Dec-9-inyl, Dodec-11-inyl, Tetradec-13-inyl, Hexadec-15-inyl, Octadec-17-inyl oder Eicos-19-inyl. Bevorzugt wird Propargyl.

$R^{10}$ und $R^{11}$ als $C_1$-$C_{12}$-Alkinyl bedeuten beispielsweise Ethinyl, Propargyl, But-3-inyl, Hex-5-inyl, Oct-7-inyl, Dec-9-inyl oder Dodec-11-inyl. Bevorzugt wird Propargyl.

$R^1$, $R^2$, $R^3$, $R^4$, $R^5$, $R^9$, $R^{10}$, $R^{11}$ oder $R^{15}$ als $C_5$-$C_{12}$-Cycloalkyl bedeuten beispielsweise Cyclopentyl, Cyclohexyl, Cycloheptyl, Cyclooctyl, Cyclononyl, Cyclodecyl, Cycloundecyl oder Cyclododecyl. Diese Reste sind bevorzugt $C_5$-$C_9$-Cycloalkyl und insbesondere Cyclohexyl.

Als $C_7$-$C_{14}$-Aralkyl bedeuten $R^1$, $R^2$, $R^3$, $R^4$, $R^5$, $R^9$, $R^{10}$, $R^{11}$ oder $R^{15}$ beispielsweise Benzyl, Phenylethyl, α-Methylbenzyl, α,α-Dimethylbenzyl, Phenylbutyl, Phenyl-α,α-dimethylpropyl, Phenylhexyl, Phenyl-α,α-dimethylbutyl, Phenyloctyl oder Phenyl-α,α-dimethylhexyl. Bevorzugt wird Benzyl.

$R^1$, $R^2$, $R^3$, $R^4$, $R^5$, $R^9$, $R^{10}$, $R^{11}$ und $R^{15}$ als $C_7$-$C_{14}$-Alkaryl sind beispielsweise o-, m- oder p-Tolyl, 2,3-, 2,4-, 2,5-, 2,6-, 3,4-, 3,5- oder 3,6-Dimethylphenyl, o-, m- oder p-Ethylphenyl, o-, m- oder p-Propylphenyl, o-, m- oder p-Isopropylphenyl, o-, m- oder p-Butylphenyl, o-, m- oder p-sek.-Butylphenyl, o-, m- oder p-tert.-Butylphenyl, 2,3-, 2,4-, 2,5-, 2,6-, 3,4-, 3,5- oder 3,6-Dibutylphenyl bzw. -Di-tert.-butylphenyl oder o-, m- oder p-Hexylphenyl oder o-, m- oder p-Octylphenyl. Bevorzugt werden o-, m- oder p-Tolyl.

$R^2$, $R^3$, $R^4$ oder $R^5$ als durch einen Phenylrest und/oder eine oder zwei Hydroxygruppen substituiertes $C_2$-$C_{20}$-Alkyl bedeuten beispielsweise 2-Hydroxyethyl, 2-Hydroxypropyl, 2-Hydroxybutyl, 2-Hydroxyhexyl, 2-Hydroxyoctyl, 2-Hydroxydecyl, 2-Hydroxydodecyl, 2-Hydroxytetradecyl, 2-Hydroxyhexadecyl, 2-Hydroxyoctadecyl, 2-Hydroxyeicosyl, 1-Phenyl-2-hydroxyethyl, 1-Phenyl-2-hydroxypropyl, 1-Phenyl-2-hydroxybutyl, 1-Phenyl-2-hydroxyhexyl, 1-Phenyl-2-hydroxyoctyl, 1-Phenyl-2-hydroxydecyl, 1-Phenyl-2-hydroxydodecyl, 1-Phenyl-2-hydroxytetradecyl, 1-Phenyl-2-hydroxyhexadecyl, 1-Phenyl-2-hydroxyoctadecyl, 1-Phenyl-2-hydroxyeicosyl oder 2,3-Dihydroxypropyl. Bevorzugt wird $C_2$-$C_{14}$-Alkyl, das durch einen Phenylrest und/oder eine oder zwei Hydroxygruppen substituiert ist. Ganz besonders bevorzugt werden 2-Hydroxyethyl, 2-Hydroxypropyl, 2,3-Dihydroxypropyl oder 1-Phenyl-2-hydroxyethyl.

$R^2$, $R^3$, $R^4$ oder $R^5$ als durch Hydroxy in 2-Position substituiertes $C_5$-$C_7$-Cycloalkyl bedeuten beispielsweise 2-Hydroxycyclopentyl, 2-Hydroxycyclohexyl oder 2-Hydroxycycloheptyl. Besonders bevorzugt wird 2-Hydroxycyclohexyl.

$R^{10}$ und $R^{11}$ als durch eine Hydroxy- oder Cyangruppe substituiertes $C_2$-$C_{20}$-Alkyl bedeuten beispielsweise 2-Hydroxyethyl, 2-Hydroxypropyl, 2-Hydroxybutyl, 2-Hydroxyhexyl, 2-Hydroxyoctyl, 2-Hydroxydecyl, 2-Hydroxydodecyl, 2-Hydroxytetradecyl, 2-Hydroxyhexadecyl, 2-Hydroxyoctadecyl, 2-Hydroxyeicosyl oder 2-Cyanethyl. Bevorzugt werden 2-Hydroxyethyl, 2-Hydroxypropyl oder 2-Cyanethyl.

$R^{10}$ und $R^{11}$ als durch ein bis fünf —O—, —S—, —$N(CH_3)$— oder —$N(C_2H_5)$-unterbrochenes gegebenenfalls mit einer Hydroxygruppe substituiertes $C_3$-$C_{20}$-Alkyl bedeuten beispielsweise 3-Oxabutyl, 3,6-Dioxaheptyl, 3,6,9-Trioxadecyl, 3,6,9,12-Tetraoxatridecyl, 3,6,9,12,15-Pentaoxahexadecyl, 3,6,9,12,15,18-Hexaoxanonadecyl, 5-Hydroxy-3-oxapentyl, 8-Hydroxy-3,6-dioxaoctyl, 11-Hydroxy-3,6,9-trioxaundecyl, 14-Hydroxy-3,6,9,12-tetraoxatetradecyl, 3-Thiabutyl, 5-Hydroxy-3-azamethylpentyl, 5-Hydroxy-3-azaethylpentyl, 3-Azamethyl-6-oxaheptyl, 3-Azaethyl-6-oxaheptyl, 3-Azamethylbutyl oder 3-Azaethylbutyl.

Bevorzugt werden durch —O— unterbrochene $C_3$-$C_{12}$-Alkylreste, die gegebenenfalls mit einer Hydroxygruppe substituiert sind. Ganz besonders bevorzugt werden 5-Hydroxy-3-oxapentyl oder 3-Oxabutyl.

Als durch ein oder zwei —$NO_2$, —Cl, —Br, —$OCH_3$ oder —$COOR^{18}$ substituiertes Phenyl bedeuten $R^{10}$ oder $R^{11}$ beispielsweise o-, m- oder p-Nitrophenyl, -Chlorphenyl, -Bromphenyl, -Methoxyphenyl, -Methoxycarbonylphenyl, -Ethoxycarbonylphenyl, 2,3-, 2,4-, 2,5-, 2,6-, 3,4- oder 3,5-Dinitrophenyl, -Dichlorphenyl, -Dibromphenyl, -Dimethoxyphenyl, -Dimethoxycarbonylphenyl oder -Diethoxycarbonylphenyl.

Bevorzugt werden o-, m- oder p-Chlorphenyl, -Methoxyphenyl oder -Methoxycarbonylphenyl. Bilden $R^{10}$ und $R^{11}$ zusammen mit dem gemeinsamen Stickstoffatom einen fünf-, sechs- oder siebengliedrigen heterozyklischen Ring, der gegebenenfalls noch ein weiteres Heteroatom enthalten kann, so handelt es sich bei diesem Ring beispielsweise um Pyrrol, 2-H-Pyrrol, Imidazol, Pyrazol, Hexamethylenimin, Pyrrolidin, Pyrrolin, Imidazolidin, Imidazolin, Pyrazolidin, Pyrazolin, Piperidin, Piperazin oder Morpholin. Bevorzugt werden Pyrrol, Pyrrolidin, Piperidin oder Morpholin und ganz besonders Piperidin oder Morpholin.

X bedeutet vorzugsweise —$C(R^{16}R^{17})$— oder —S— und besonders bevorzugt —$C(R^{16}R^{17})$—. $R^{16}$ und $R^{17}$ sind bevorzugt Wasserstoff oder $C_1$-$C_6$-Alkyl. Ganz besonders bevorzugte Reste X sind —$CH_2$—, —$CH(CH_3)$— oder —$C(CH_3)_2$—.

Als Beispiele für Vertreter von Verbindungen der Formel I sind die nachfolgend aufgezählten Substanzen anzusehen :

2,4-Bis-(methylthiomethyl)-6-tert.-butylphenol,
2,4-Bis-(ethylthiomethyl)-6-tert.-butylphenol,
2,4-Bis-(n-propylthiomethyl)-6-tert.-butylphenol,
2,4-Bis-(n-butylthiomethyl)-6-tert.-butylphenol,
2,4-Bis-(n-hexylthiomethyl)-6-tert.-butylphenol,
2,4-Bis-(n-octylthiomethyl)-6-tert.-butylphenol,
2,4-Bis-(n-decylthiomethyl)-6-tert.-butylphenol,
2,4-Bis-(n-dodecylthiomethyl)-6-tert.-butylphenol,
2,4-Bis-(n-tetradecylthiomethyl)-6-tert.-butylphenol,
2,4-Bis-(n-hexadecylthiomethyl)-6-tert.-butylphenol,
2,4-Bis-(n-octadecylthiomethyl)-6-tert.-butylphenol,
2,4-Bis-(n-eicosylthiomethyl)-6-tert.-butylphenol,
2,4-Bis-(isopropylthiomethyl)-6-tert.-butylphenol,
2,4-Bis-(sec-butylthiomethyl)-6-tert.-butylphenol,
2,4-Bis-(tert.-butylthiomethyl)-6-tert.-butylphenol,
2,4-Bis-(2-ethylhexylthiomethyl)-6-tert.-butylphenol,
2,4-Bis-(1,1,3,3-tetramethylbutylthiomethyl)-6-tert.-butylphenol,
2,4-Bis-(1,1,3,3,5,5-hexamethylhexylthiomethyl)-6-tert.-butylphenol,
2,4-Bis-(n-octylthiomethyl)-6-isopropylphenol,
2,4-Bis-(n-dodecylthiomethyl)-6-isopropylphenol,
2,4-Bis-(n-octylthiomethyl)-6-(2-ethylhexyl)-phenol,
2,4-Bis-(n-dodecylthiomethyl)-6-(2-ethylhexyl)-phenol,
2,4-Bis-[4-(2,2,4,6,6-pentamethylheptyl)-thiomethyl]-6-tert.-butylphenol,
2,4-Bis-(n-octylthiomethyl)-6-(1,1-dimethylbutyl)-phenol,
2,4-Bis-(n-dodecylthiomethyl)-6-(1,1-dimethylbutyl)-phenol,
2,4-Bis-(n-dodecylthiomethyl)-6-(1,1-dimethylpropyl)-phenol,
2,4-Bis-(n-octylthiomethyl)-6-cyclohexylphenol,
2,4-Bis-(n-dodecylthiomethyl)-6-cyclohexylphenol,
2,4-Bis-(n-octylthiomethyl)-6-phenylphenol,
2,4-Bis-(n-dodecylthiomethyl)-6-phenylphenol,
2,4-Bis-(n-octylthiomethyl)-6-benzylphenol,
2,4-Bis-(n-dodecylthiomethyl)-6-benzylphenol,
2,4-Bis-(n-dodecylthiomethyl)-6-($\alpha$,$\alpha$-dimethylbenzyl)-phenol,
2,4-Bis-(n-octylthiomethyl)-6-p-tolylphenol,
2,4-Bis-(n-dodecylthiomethyl)-6-p-tolylphenol,
2,4-Bis-(n-octylthiomethyl)-6-prop-2-enylphenol,
2,4-Bis-(n-dodecylthiomethyl)-6-prop-2-enylphenol,
2,4-Bis-(n-dodecylthiomethyl)-6-prop-2-inylphenol,
2,4-Bis-(prop-2-enylthiomethyl)-6-tert.-butylphenol,
2,4-Bis-(prop-2-inylthiomethyl)-6-tert.-butylphenol,
2,4-Bis-(cyclohexylthiomethyl)-6-tert.-butylphenol,
2,4-Bis-(2-hydroxycyclohexylthiomethyl)-6-tert.-butylphenol,
2,4-Bis-(phenylthiomethyl)-6-tert.-butylphenol,
2,4-Bis-(benzylthiomethyl)-6-tert.-butylphenol,

2,4-Bis-(p-tolylthiomethyl)-6-tert.-butylphenol,
Dimethylester des 2,4-Bis-(3-carboxy-2-thiapropyl)-6-tert.-butylphenols,
2,4-Bis-(3-carboxy-2-thiapropyl)-6-tert.-butylphenol,
Dibutylester des 2,4-Bis-(3-carboxy-2-thiapropyl)-6-tert.-butylphenols,
Dioctylester des 2,4-Bis-(3-carboxy-2-thiapropyl)-6-tert.-butylphenols,
Didodecylester des 2,4-Bis-(3-carboxy-2-thiapropyl)-6-tert.-butylphenols,
Monomethylester des 2,4-Bis-(3-carboxy-2-thiapropyl)-6-tert.-butylphenols,
Dimethylester des 2,4-Bis-(4-carboxy-2-thiabutyl)-6-tert.-butyl phenols,
Dioctylester des 2,4-Bis-(4-carboxy-2-thiabutyl)-6-tert.-butylphenols,
Di-(2-ethylhexyl)-ester des 2,4-Bis-(3-carboxy-2-thiapropyl)-6-tert.-butylphenols,
Dimethylester des 2,4-Bis-(3-carboxy-2-thiabutyl)-6-tert.-butylphenols,
Dimethylester des 2,4-Bis-(4-carboxy-3-methyl-2-thiapentyl)-6-tert.-butylphenols,
N,N-Dimethylamid des 2,4-Bis-(3-carboxy-2-thiapropyl)-6-tert.-butylphenols,
N,N-Dihexylamid des 2,4-Bis-(3-carboxy-2-thiapropyl)-6-tert.-butylphenols,
N,N-Didodecylamid des 2,4-Bis-(3-carboxy-2-thiapropyl)-6-tert.-butylphenols,
N,N-Dimethylamid des 2,4-Bis-(4-carboxy-2-thiabutyl)-6-tert.-butylphenols,
N,N-Dimethylamid des 2,4-Bis-[3-carboxy-2-thiabutyl]-6-tert.-butylphenols,
N,N-Dibutylamid des 2,4-Bis-(4-carboxy-3-methyl-2-thiapentyl)-6-tert.-butylphenols,
Dicyclohexylester des 2,4-Bis-(3-carboxy-2-thiapropyl)-6-tert.-butylphenols,
Diphenylester des 2,4-Bis-(3-carboxy-2-thiapropyl)-6-tert.-butylphenols,
Dibenzylester des 2,4-Bis-(3-carboxy-2-thiapropyl)-6-tert.-butylphenols,
Di-p-tolylester des 2,4-Bis-(3-carboxy-2-thiapropyl)-6-tert.-butylphenols,
Di-(3-thiabutyl)-ester des 2,4-Bis-(3-carboxy-2-thiapropyl)-6-tert.-butylphenols,
Di-(3-oxabutyl)-ester des 2,4-Bis-(3-carboxy-2-thiapropyl)-6-tert.-butylphenols,
Di-(N,N-dimethylamino-2-ethyl)-ester des 2,4-Bis-(3-carboxy-2-thiapropyl)-6-tert.-butylphenols,
Diamid des 2,4-Bis-(3-carboxy-2-thiapropyl)-6-tert.-butylphenols,
Diamid des 2,4-Bis-(4-carboxy-2-thiabutyl)-6-tert.-butylphenols,
2,4-Bis-(prop-2-enylthiomethyl)-6-tert.-butylphenol,
2,4-Bis-(prop-2-inylthiomethyl)-6-tert.-butylphenol,
2,4-Bis-[2-hydroxyethylthiomethyl]-6-tert.-butylphenol,
2,4-Bis-[2-cyanoethylthiomethyl]-6-tert.-butylphenol,
2,4-Bis-[(4-methoxyphenyl)-thiomethyl]-6-tert.-butylphenol,
2,4-Bis-[(4-chlorphenyl)-thiomethyl]-6-tert.-butylphenol,
2,4-Bis-[(2-methoxycarbonylphenyl)-thiomethyl]-6-tert.-butylphenol,
2,4-Bis-[(1,3-benzthiazol-2-yl)-thiomethyl]-6-tert.-butylphenol,
2,4-Bis-[2,3-dihydroxypropylthiomethyl]-6-tert.-butylphenol,
2,4-Bis-[(3,5-di-tert.-butyl-4-hydroxyphenyl)-thiomethyl]-6-tert.-butylphenol,
2,4-Bis-[4-(3,5-di-tert.-butyl-4-hydroxyphenyl)-2-thiabutyl]-6-tert.-butylphenol,
2,4-Bis-[4-acetyloxy-2-thiabutyl]-6-tert.-butylphenol,
2,4-Bis-[3-formyl-2-thiabutyl]-6-tert.-butylphenol,
2,4-Bis-[3-acetyl-2-thiabutyl]-6-tert.-butylphenol.

Die Herstellung der Verbindungen der Formel I erfolgt nach an sich bekannten Verfahren. So setzt man beispielsweise in Analogie zur Beschreibung in der GB-PS 1,184,533 einen Molanteil eines geeignet o-substituierten Phenols oder eines 2,2'-Bisphenols mit zwei bzw. vier Molanteilen Formaldehyd und den entsprechenden Mengen geeignet substituierter Merkaptane um. Es lassen sich auch Gemische unterschiedlicher Merkaptane verwenden.

Die Reaktion lässt sich in An- oder in Abwesenheit eines organischen Lösungsmittels in Gegenwart eines basischen Katalysators durchführen.

Geeignete Lösungsmittel sind Alkohole mit ein bis sechs Kohlenstoffatomen, wie beispielsweise Methanol, Ethanol, Propanol, Butanol, Pentanol oder Hexanol. Man kann aber auch Diole, Polyole sowie deren Ether verwenden, wie beispielsweise Glycol, Glycerin oder Polyethylenglycol. Die Reaktion lässt sich auch in polaren aprotischen Lösungsmitteln, wie beispielsweise Dimethylformamid oder Dimethylsulfoxid durchführen, oder es können aromatische oder aliphatische gegebenenfalls chlorierte Kohlenwasserstoffe wie beispielsweise Toluol, Ligroin oder Chlorbenzol eingesetzt werden. Als basische Katalysatoren verwendet man beispielsweise organische Basen, wie Dialkylamine oder Trialkylamine, oder man nimmt anorganische Basen, wie Hydroxide, vorzugsweise Alkalihydroxide. Anorganische Basen setzt man allerdings bevorzugt nur dann ein, wenn die Reaktanden keine hydrolisierbaren Gruppen, wie beispielsweise Ester- oder Amidgruppen enthalten.

Anstelle des Formaldehyds können auch Verbindungen, die unter Reaktionsbedingungen Formaldehyd bilden, eingesetzt werden. Dazu zählen beispielsweise Paraformaldehyd oder Hexamethylentetramin.

Das Reaktionsgemisch wird während 5 bis 40 Stunden in einer Stickstoffatmosphäre unter Rückfluss erhitzt.

Nach dem Abkühlen auf Raumtemperatur wird die organische Phase mit einem geeigneten Lösungsmittel, beispielsweise mit Toluol, Chloroform, Methylenchlorid, Ether oder Methylisobutylketon,

verdünnt. Anschliessend wird mit wässriger Säure neutral gewaschen. Dazu lässt sich beispielsweise Essigsäure einsetzen, aber es kann auch jede beliebige andere Säure, beispielsweise eine Mineralsäure genommen werden.

Die organische Phase wird nach der üblichen Abtrennung im Vakuum konzentriert und der Eindampfrückstand gegebenenfalls weiter gereinigt, beispielsweise durch Umkristallisieren, durch Säulenchromatographie oder durch Filtration über eine kurze Kieselgelsäule.

Die Verbindungen der Formel I lassen sich aber auch in Analogie zu dem in der US-PS 4,091,037 beschriebenen Verfahren durch Umsetzung einer geeigneten Mannichbase und mindestens der stöchiometrischen Menge eines geeigneten Merkaptans in An- oder Abwesenheit eines geeigneten organischen Lösungsmittels unter einer Stickstoffatmosphäre synthetisieren. Es lassen sich auch Gemische von Merkaptanen einsetzen. Die Reaktionstemperatur liegt zwischen 100 °C und 160 °C. Es eignen sich die gleichen organischen Lösungsmittel wie für die oben beschriebene Variante. Die Reaktion kann durch Anlegen eines leichten Vakuums beschleunigt werden (0,1 bis 0,6 bar). Die Aufarbeitung erfolgt wie bereits weiter oben beschrieben.

In einer weiteren Variante des Herstellungsverfahrens von Verbindungen der Formel I geht man von geeignet substituierten Chlormethylphenolen aus. Diese werden mit mindestens der äquivalenten Menge Merkaptan bzw. Merkaptid reagieren gelassen. Eventuell setzt man eine dem Merkaptan äquivalente Menge einer Base zu. Die Base kann während oder nach der Reaktion zugegeben werden. Sie dient der Neutralisation des freigesetzten Chlorwasserstoffs. Geeignete Basen sind Trialkylamine, Pyridine, Kaliumcarbonat bzw. Alkali- oder Erdalkalihydroxide.

Die als Ausgangsprodukte einzusetzenden o-substituierten Phenole, Chlormethylphenole oder Mannich-Basen sind bekannt oder können durch bekannte Verfahren hergestellt werden. So werden 2,4-Bis-(dialkylaminomethyl)-6-tert.-butylphenole in C.A. 97, 41279 beschrieben während 2,4-Bis-(chlormethyl)-6-methylphenole in C.A. 95, 669 zitiert sind.

Die Erfindung betrifft weiterhin Zusammensetzungen enthaltend ein gegen thermischen, oxidativen oder strahlungsinduzierten Abbau empfindliches organisches Material und mindestens eine Verbindung der Formel I, bevorzugt eine Verbindung der Formel I, worin $R^1$ tert.Butyl, Cyclohexyl, Phenyl oder Benzyl ist, Y $—CH_2—S—R^2$ bedeutet, Z $—CH_2—S—R^3$ ist, und worin $R^2$ und $R^3$ unabhängig voneinander $C_4$-$C_{14}$Alkyl, 2-Hydroxyethyl, 2,3-Dihydroxypropyl, Phenyl oder Benzyl sind. Auch von Interesse sind Zusammensetzungen enthaltend die oben als bevorzugt erwähnten Verbindungen der Formel I, worin $R^2$ und $R^3$ unabhängig voneinander $C_4$-$C_{14}$Alkyl sind.

Weiterhin bevorzugt werden Zusammensetzungen enthaltend ein gegen thermischen, oxidativen oder strahlungsinduzierten Abbau empfindliches organisches Material und mindestens eine Verbindung der Formel I, worin $R^1$ tert.Butyl, Cyclohexyl, Phenyl oder Benzyl bedeutet, worin Y $—CH_2—S—R^2$ ist, Z $—CH_2—S—R^3$ bedeutet, und worin $R^2$ und $R^3$ $—(CH_2)_{m+1}—W$ sind, worin m 0, 1 oder 2 ist, und worin W $—COOR^{10}$ oder $—CON(R^{10}R^{11})$ ist, wobei $R^{10}$ und $R^{11}$ unabhängig voneinander Wasserstoff, $C_1$-$C_{12}$Alkyl, Phenyl, Cyclohexyl, Benzyl oder durch —O— unterbrochenes $C_3$-$C_{12}$Alkyl bedeuten.

In einer bevorzugten Ausführungsform enthalten die erfindungsgemässen Zusammentsetzungen Gemische von Verbindungen der Formel I. Bevorzugt werden weiterhin Zusammensetzungen, worin das organische Material ein Polymeres, insbesondere ein Elastomeres, bedeutet. Als Elastomere werden insbesondere bevorzugt :

Polydiene, wie beispielsweise Polybutadien, Polyisopren oder Polychloropren ; Blockpolymere, wie beispielsweise Styrol/Butadien/Styrol, Styrol/Isopren/Styrol oder Styrol/Ethylen-Propylen/Styroltypen ; sowie Acrylnitril/Butadien Polymere.

Diese Polymere können auch in Form von Latices vorliegen und können als solche stabilisiert sein.

Ebenfalls bevorzugt werden Zusammensetzungen, worin das organische Material ein synthetischer Schmierstoff oder ein Schmierstoff auf Mineralölbasis ist.

Die in Frage kommenden Schmierstoffe sind dem Fachmanm Geläufig und z. B. im « Schmiermittel Taschenbuch (Hüthig Verlag, Heidelberg, 1974) » beschrieben.

Ein weiterer Gegenstand der Erdindung ist die Verwendung von Verbindungen der Formel I als Stabilisatoren für organisches Material gegen dessen Schädigung durch Einwirkung von Sauerstoff, Wärme, Licht und energiereiche Strahlung.

Bevorzugt ist die Verwendung der Verbindungen als Antioxidantien in organischen Polymeren besonders in Elastomeren, oder die Verwendung in Mineralölen oder synthetischen Oelen.

Die erfindungsgemässen Verbindungen eignen sich auch als EP/AW Zusätze für Schmierstoffe oder als Zusätze für Metallbearbeitungsflüssigkeiten.

Weitere Beispiele für organisches Material, welches mit den erfindungsgemässen Verbindungen vorteilhaft stabilisert werden kann, sind :

1. Polymere von Mono- und Diolefinen, beispielsweise Polyethylen (das gegebenenfalls vernetzt sein kann), Polypropylen, Polyisobutylen, Polybuten-1, Polymethylpenten-1, Polyisopren oder Polybutadien sowie Polymerisate von Cycloolefinen wie z. B. von Cyclopenten oder Norbornen.

2. Mischungen der unter 1) genannten Polymeren, z. B. Mischungen von Polypropylen mit Polyisobutylen.

3. Copolymere von Mono- und Diolefinen untereinander oder mit anderen Vinylmonomeren, wie z. B. Ethylen-Propylen-Copolymere, Propylen-Buten-1-Copolymere, Propylen-Isobutylen-Copolymere, Ethylen-Buten-1-Copolymere, Propylen-Butadien-Copolymere, Isobutylen-Isopren-Copolymere, Ethylen-Alkylacrylat-Copolymere, Ethylen-Alkylmethacrylat-Copolymere, Ethylen-Vinylacetat-Copolymere oder Ethylen-Acrylsäure-Copolymere und deren Salze (Ionomere), sowie Terpolymere von Ethylen mit Propylen und einem Dien, wie Hexadien, Dicyclopentadien oder Aethylidennorbornen.

4. Polystyrol, Poly-(p-methylstyrol).

5. Copolymere von Styrol oder α-Methylstyrol mit Dienen oder Acrylderivaten, wie z. B. Styrol-Butadien, Styrol-Acrylnitril, Styrol-Alkylmethacrylat, Styrol-Maleinanhydrid, Styrol-Acrylnitril-Methylacrylat ; Mischungen von hoher Schlagzähigkeit aus Styrol-Copolymeren und einem anderen Polymer wie z. B. einem Polyacrylat, einem Dien-Polymeren oder einem Ethylen-Propylen-Dien-Terpolymeren ; sowie Block-Copolymere des Styrols, wie z. B. Styrol-Butadien-Styrol, Styrol-Isopren-Styrol, Styrol-Ethylen-/Butylen-Styrol oder Styrol-Ethylen/Propylen-Styrol.

6. Pfropfcopolymere von Styrol, wie z. B. Styrol auf Polybutadien, Styrol und Acrylnitril auf Polybutadien, Styrol und Maleinsäureanhydrid auf Polybutadien, Styrol und Alkylacrylate bzw. Alkylmethacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 5) genannten Copolymeren, wie sie z. B. als sogenannte, ABS, MBS, ASA oder AES-Polymere bekannt sind.

7. Halogenhaltige Polymere, wie z. B. Polychloropren, Chlorkautschuk, chloriertes oder chlorsulfoniertes Polyethylen, Epichlorhydrinhomo- und -copolymere, insbesondere Polymere aus halogenhaltigen Vinylverbindungen, wie z. B. Polyvinylchlorid, Polyvinylidenchlorid, Polyvinylidenfluorid ; sowie deren Copolymere wie Vinylchlorid-Vinylidenchlorid, Vinylchlorid-Vinylacetat oder Vinylidenchlorid-Vinylacetat.

8. Polymere, die sich von α,β-ungesättigten Säuren und deren Derivaten ableiten, wie Polyacrylate und Polymethacrylate, Polyacrylamide und Polyacrylnitrile.

9. Copolymere der unter 8) genannten Monomeren untereinander oder mit anderen ungesättigten Monomeren, wie z. B. Acrylnitril-Butadien-Copolymere, Acrylnitril-Alkylacrylat-Copolymere, Acrylnitril-Alkoxyalkylacrylat-Copolymere, Acrylnitril-Vinylhalogenid-Copolymere oder Acrylnitryl-Alkylmethacrylat-Butadien-Terpolymere.

10. Polymere, die sich von ungesättigten Alkoholen und Aminen bzw. deren Acylderivaten oder Acetalen ableiten, wie Polyvinylalkohol, Polyvinylacetat-, -stearat, -benzoat, -maleat, Polyvinylbutyral, Polyallylphthalat, Polyallylmelamin.

11. Homo- und Copolymere von cyclischen Ethern, wie Polyalkylenglykole, Polyethylenoxyd, Polypropylenoxyd oder deren Copolymere mit Bisglycidylethern.

12. Polyacetale, wie Polyoxymethylen, sowie solche Polyoxymethylene, die Comonomere wie z. B. Ethylenoxid enthalten.

13. Polyphenyloxide und -sulfide und deren Mischungen mit Styrolpolymeren.

14. Polyurethane, die sich von Polyethern, Polyestern und Polybutadienen mit endständigen Hydroxylgruppen einerseits und aliphatischen oder aromatischen Polyisocyanaten andererseits ableiten sowie deren Vorprodukte.

15. Polyamide und Copolyamide, die sich von Diaminen und Dicarbonsäuren und/oder von Aminocarbonsäuren oder den entsprechenden Lactamen ableiten, wie Polyamid 4, Polyamid 6, Polyamid 6/6, Polyamid 6/10, Polyamid 11, Polyamid 12, Poly-2,4,4-trimethylhexamethylenterephthalamid, Poly-m-phenylen-isophthalamid, sowie deren Block-Copolymere mit Polyethern wie z. B. mit Polyethylenglykol, Polypropylenglykol oder Polytetramethylenglykol.

16. Polyharnstoffe, Polyimide, Polyamid-imide und Polybenzimidazole.

17. Polyester, die sich von Dicarbonsäuren und Dialkoholen und/oder von Hydroxycarbonsäuren oder den entsprechenden Lactonen ableiten, wie Polyethylenterephthalat, Polybutylenterephthalat, Poly-1,4-dimethylolcyclohexanterephthalat, Polyhydroxybenzoate, sowie Block-Polyether-ester, die sich von Polyethern mit Hydroxylendgruppen ableiten.

18. Polycarbonate und Polyestercarbonate.

19. Polysulfone, Polyethersulfone und Polyetherketone.

20. Vernetzte Polymere, die sich von Aldehyden einerseits und Phenolen, Harnstoff oder Melamin andererseits ableiten, wie Phenol-Formaldehyd-, Harnstoff-Formaldehyd- und Melamin-Formaldehydharze.

21. Trocknende und nicht-trocknende Alkydharze.

22. Ungesättigte Polyesterharze, die sich von Copolyestern gesättigter und ungesättigter Dicarbonsäuren mit mehrwertigen Alkoholen, sowie Vinylverbindungen als Vernetzungsmittel ableiten, wie auch deren halogenhaltige, schwerbrennbare Modifikationen.

23. Vernetzbare Acrylharze, die sich von substituierten Acrylsäureestern ableiten wie z. B. von Epoxyacrylaten, Urethan-acrylaten oder Polyester-acrylaten.

24. Alkydharze, Polyesterharze und Acrylatharze, die mit Melaminharzen, Harnstoffharzen, Polyisocyanaten oder Epoxidharzen vernetzt sind.

25. Vernetzte Epoxidharze, die sich von Polyepoxiden ableiten, z. B. von Bis-glycidylethern oder von cycloaliphatischen Diepoxiden.

26. Natürliche Polymere, wie Cellulose, Naturkautschuk, Gelatine, sowie deren polymerhomolog

chemisch abgewandelte Derivate, wie Celluloseacetate, -propionate und -butyrate, bzw. die Celluloseether, wie Methylcellulose.

27. Mischungen (Polyblends) der vorgenannten Polymeren wie z. B. PP/EPDM, Polyamid 6/EPDM oder ABS, PVC/EVA, PVC/ABS, PVC/MBS, PC/ABS, PBTP/ABS.

28. Natürliche und synthetische organische Stoffe, die reine monomere Verbindungen oder Mischungen von solchen darstellen, beispielsweise Mineralöle, tierische oder pflanzliche Fette, Oele und Wachse, oder Oele, Wachse und Fette auf Basis synthetischer Ester (z. B. Phthalate, Adipate, Phosphate oder Trimellithate), sowie Abmischungen synthetischer Ester mit Mineralölen in beliebigen Gewichtsverhältnissen, wie sie z. B. als Spinnpräparationen Anwendung finden, sowie deren wässrigen Emulsionen.

29. Wässrige Emulsionen natürlicher oder synthetischer Kautschuke, wie z. B. Naturkautschuk-Latex oder Latices von carboxylierten Styrol-Butadien-Copolymeren.

Die Stabilisatoren werden den Kunststoffen bzw. den Schmiermitteln in einer Konzentration von 0.01-10 Gew.-% berechnet auf das zu stabilisierende Material zugesetzt. Vorzugsweise werden 0,05 bis 5,0 Gew.-%, besonders bevorzugt 0,1 bis 2,0 Gew.-% der Verbindungen, berechnet auf das zu stabilisierende Material, in dieses eingearbeitet.

Die Einarbeitung kann beispielsweise durch Einmischen der Substanzen der Formel I und gegebenenfalls weiterer Additive nach den in der Technik üblichen Methoden, vor oder während der Formgebung, oder auch durch Aufbringen der gelösten oder dispergierten Verbindungen auf das Polymere, gegebenenfalls unter nachträglichem Verdunsten des Lösungsmittels erfolgen. Die neuen Verbindungen können auch in Form eines Masterbatches, der diese Verbindungen beispielsweise in einer Konzentration von 2,5 bis 25 Gew.-% enthält, den zu stabilisierenden Kunststoffen zugesetzt werden.

Im Falle von vernetztem Polyethylen werden die Verbindungen vor der Vernetzung beigefügt.

Die so stabilisierten Materialien können in verschiedenster Form angewendet werden, z. B. als Folien, Fasern, Bändchen, Formmassen, Profile oder als Bindemittel für Lacke, Klebstoffe oder Kitte.

In der Praxis können die Phenole der Formel I zusammen mit anderen Stabilisatoren eingesetzt werden.

Schmierstoff-Formulierungen können zusätzlich noch andere Additive enthalten, die zugegeben werden, um gewisse Gebrauchseigenschaften zu verbessern, wie z. B. weitere aminische Antioxidantien, Metallpassivatoren, Rostinhibitoren, Viskositäts-Index-Verbesserer, Stockpunkterniedriger, Dispergiermittel/Tenside und Verschleisschutzadditive.

Als weitere Additive, mit denen zusammen die erfindungsgemäss verwendeten Stabilisatoren eingesetzt werden können, sind beispielsweise zu nennen :

1. Antioxidantien

1.1. Alkylierte Monophenole
2,6-Di-tert.butyl-4-methylphenol
2-Tert.butyl-4,6-dimethylphenol
2,6-Di-tert.butyl-4-ethylphenol
2,6-Di-tert.butyl-4-n-butylphenol
2,6-Di-tert.butyl-4-i-butylphenol
2,6-Di-cyclopentyl-4-methylphenol
2-(α-Methylcyclohexyl)-4,6-dimethylphenol
2,6-Di-octadecyl-4-methylphenol
2,4,6-Tri-cyclohexylphenol
2,6-Di-tert.butyl-4-methoxymethylphenol

1.2. Alkylierte Hydrochinone
2,6-Di-tert.butyl-4-methoxyphenol
2,5-Di-tert.butyl-hydrochinon
2,5-Di-tert.amyl-hydrochinon
2,6-Diphenyl-4-octadecyloxyphenol

1.3. Hydroxylierte Thiodiphenylether
2,2'-Thio-bis-(6-tert.butyl-4-methylphenol)
2,2'-Thio-bis-(4-octylphenol)
4,4'-Thio-bis-(6-tert.butyl-3-methylphenol)
4,4'-Thio-bis-(6-tert.butyl-2-methylphenol)

1.4. Alkyliden-Bisphenole
2,2'-Methylen-bis-(6-tert.butyl-4-methylphenol)
2,2'-Methylen-bis-(6-tert.butyl-4-ethylphenol)
2,2'-Methylen-bis-[4-methyl-6-(α-methylcyclohexyl)-phenol]
2,2'-Methylen-bis-(4-methyl-6-cyclohexylphenol)

2,2'-Methylen-bis-(6-nonyl-4-methylphenol)
2,2'-Methylen-bis-(4,6-di-tert.butylphenol)
2,2'-Ethyliden-bis-(4,6-di-tert.butylphenol)
2,2'-Ethyliden-bis-(6-tert.butyl-4-isobutylphenol)
2,2'-Methylen-bis-[6-(α-methylbenzyl)-4-nonylphenol]
2,2'-Methylen-bis-[6-(α,α-dimethylbenzyl)-4-nonylphenol]
4,4'-Methylen-bis-(2,6-di-tert.butylphenol)
4,4'-Methylen-bis-(6-tert.butyl-2-methylphenol)
1,1-Bis-(5-tert.butyl-4-hydroxy-2-methylphenyl)-butan
2,6-Di-(3-tert.butyl-5-methyl-2-hydroxybenzyl)-4-methylphenol
1,1,3-Tris-(5-tert.butyl-4-hydroxy-2-methylphenyl)-butan
1,1-Bis-(5-tert.butyl-4-hydroxy-2-methylphenyl)-3-n-dodecylmercaptobutan
Ethylenglycol-bis-[3,3-bis-(3'-tert.butyl-4'-hydroxyphenyl)-butyrat]
Di-(3-tert.butyl-4-hydroxy-5-methylphenyl)-dicyclopentadien
Di-[2-(3'-tert.butyl-2'-hydroxy-5'-methyl-benzyl)-6-tert.butyl-4-methyl-phenyl]-terephthalat.

1.5. Benzylverbindungen
1,3,5-Tri-(3,5-di-tert.butyl-4-hydroxybenzyl)-2,4,6-trimethylbenzol
Di-(3,5-di-tert.butyl-4-hydroxybenzyl)-sulfid
3,5-di-tert.butyl-4-hydroxybenzyl-mercaptoessigsäure-isooctylester
Bis-(4-tert.butyl-3-hydroxy-2,6-dimethylbenzyl)dithiol-terephthalat
1,3,5-Tris-(3,5-di-tert.butyl-4-hydroxybenzyl)-isocyanurat
1,3,5-Tris-(4-tert.butyl-3-hydroxy-2,6-dimethylbenzyl)-isocyanurat
3,5-Di-tert.butyl-4-hydroxybenzyl-phosphonsäure-dioctadecylester
3,5-Di-tert.butyl-4-hydroxybenzyl-phosphonsäure-monoethylester, Calcium-salz

1.6. Acylaminophenole
4-Hydroxy-laurinsäureanilid
4-Hydroxy-stearinsäureanilid
2,4-Bis-octylmercapto-6-(3,5-di-tert.butyl-4-hydroxyanilino)-s-triazin
N-(3,5-di-tert.butyl-4-hydroxyphenyl)-carbaminsäureoctylester.

1.7. Ester der β-(3,5-Di-tert.butyl-4-hydroxyphenyl)-propionsäure mit ein- oder mehrwertigen Alkoholen, wie z. B. mit

| | |
|---|---|
| Methanol | Diethylenglycol |
| Octadecanol | Triethylenglycol |
| 1,6-Hexandiol | Pentaerythrit |
| Neopentylglycol | Tris-hydroxyethyl-isocyanurat |
| Thiodiethylenglycol | Di-hydroxyethyl-oxalsäurediamid |

1.8. Ester der β-(5-tert.butyl-4-hydroxy-3-methylphenyl)-propionsäure mit ein- oder mehrwertigen Alkoholen, wie z. B. mit

| | |
|---|---|
| Methanol | Diethylenglycol |
| Octadecanol | Triethylenglycol |
| 1,6-Hexandiol | Pentaerythrit |
| Neopentylglycol | Tris-hydroxyethyl-isocyanurat |
| Thiodiethylenglycol | Di-hydroxyethyl-oxalsäurediamid |

1.9. Amide der β-(3,5-Di-tert.butyl-4-hydroxyphenyl)-propionsäure, wie z. B.
N,N'-Di-(3,5-di-tert.butyl-4-hydroxyphenylpropionyl)-hexamethylendiamin
N,N'-Di-(3,5-di-tert.butyl-4-hydroxyphenylpropionyl)-trimethylendiamin
N,N'-Di-(3,5-di-tert.butyl-4-hydroxyphenylpropionyl)-hydrazin

2. UV-Absorber und Lichtschutzmittel

2.1. 2-(2'-Hydroxyphenyl)-benztriazole, wie z. B. das 5'-Methyl-, 3',5'-Di-tert.butyl-, 5'-Tert.butyl-, 5'-(1,1,3,3-Tetramethylbutyl)-, 5-Chlor-3', 5'-di-tert.butyl-, 5-Chlor-3'-tert.butyl-5'-methyl-, 3'-sec.Butyl-5'-tert.butyl, 4'-Octoxy-, 3', 5'-Di-tert.amyl-, 3', 5'-Bis-(α,α-dimethylbenzyl)-Derivat.

2.2. 2-Hydroxybenzophenone, wie z. B. das 4-Hydroxy-, 4-Methoxy-, 4-Octoxy-, 4-Decyloxy-, 4-Dodecyloxy-, 4-Benzyloxy-, 4,2',4'-Trihydroxy-, 2'-Hydroxy-4,4'-dimethoxy-Derivat.

2.3. Ester von gegebenenfalls substituierten Benzoesäuren, wie z. B. 4-Tert.butyl-phenylsalicylat,

Phenylsalicylat, Octylphenylsalicylat, Dibenzoylresorcin, Bis-(4-tert.butylbenzoyl)-resorcin, Benzoylresorcin, 3,5-Di-tert.butyl-4-hydroxybenzoesäure-2,4-di-tert.butylphenylester, 3,5-Di-tert.butyl-4-hydroxybenzoesäurehexadecylester.

2.4. Acrylate, wie z. B. α-Cyan-β,β-diphenylacrylsäure-ethylester bzw. -isooctylester, α-Carbomethoxyzimtsäuremethylester, α-Cyano-β-methyl-p-methoxy-zimtsäuremethylester bzw. -butylester, α-Carbomethoxy-p-methoxy-zimtsäure-methylester, N-(β-Carbomethoxy-β-cyanovinyl)-2-methyl-indolin.

2.5. Nickelverbindungen, wie z. B. Nickelkomplexe des 2,2'-Thio-bis-[4-(1,1,3,3-tetramethylbutyl)-phenols], wie der 1 : 1- oder der 1 : 2-Komplex, gegebenenfalls mit zusätzlichen Liganden wie n-Butylamin, Triethanolamin oder N-Cyclohexyl-diethanolamin, Nickeldibutyl-dithiocarbamat, Nickelsalze von 4-Hydroxy-3,5-di-tert.butylbenzyl-phosphonsäure-monoalkylestern wie vom Methyl- oder Ethylester, Nickelkomplexe von Ketoximen wie von 2-Hydroxy-4-methyl-phenyl-undecylketonoxim, Nickelkomplexe des 1-Phenyl-4-lauroyl-5-hydroxy-pyrazols, gegebenenfalls mit zusätzlichen Liganden.

2.6. Sterisch gehinderte Amine, wie z. B. Bis-(2,2,6,6-tetramethyl-piperidyl)-sebacat Bis-(1,2,2,6,6,-pentamethylpiperidyl)-sebacat n-Butyl-3,5-di-tert.butyl-4-hydroxybenzyl-malonsäure-bis(1,2,2,6,6-pentamethylpiperidyl)-ester, Kondensationsprodukt aus 1-Hydroxy-ethyl-2,2,6,6-tetramethyl-4-hydroxypiperidin und Bernsteinsäure, Kondensationsprodukt aus N,N'-(2,2,6,6-Tetramethyl-4-piperidyl)-hexamethylendiamin und 4-tert.Octylamino-2,6-dichlor-1,3,5-s-triazin, Tris-(2,2,6,6-tetramethyl-4-piperidyl)-nitrilotriacetat, Tetrakis-(2,2,6,6-tetramethyl-4-piperidyl)-1,2,3,4-butantetracarbonsäure, 1,1'-(1,2-Ethandiyl)-bis-(3,3,5,5-tetramethyl-piperazinon).

2.7. Oxalsäurediamide, wie z. B. 4,4'-Di-octyloxy-oxanilid, 2,2'-Di-octyloxy-5,5'-di-tert.butyl-oxanilid, 2,2'-Di-dodecyloxy-5,5'-di-tert.butyl-oxanilid, 2-Ethoxy-2'-ethyl-oxanilid, N,N'-Bis-(3-dimethylaminopropyl)-oxalamid, 2-Ethoxy-5-tert.butyl-2'-ethyl-oxanilid und dessen Gemisch mit 2-Ethoxy-2'-ethyl-5,4'-di-tert.butyl-oxanilid, Gemische von ortho- und para-Methoxy- sowie von o- und p-Ethoxy-di-substituierte Oxaniliden.

3. Metalldesaktivatoren, wie z. B. N,N'-Diphenyloxalsäurediamid, N-Salicylal-N'-salicyloylhydrazin, N,N'-Bis-salicyloylhydrazin, N,N Bis-(3,5-di-tert.butyl-4-hydroxyphenylpropionyl)-hydrazin, 3-Salicyloylamino-1,2,4-triazol, Bis-benzyliden-oxalsäuredihydrazid.

4. Phosphite und Phosphonite, wie z. B. Triphenylphosphit, Diphenylalkylphosphite, Phenyldialkylphosphite, Tri-(nonylphenyl)-phosphit, Trilaurylphosphit, Trioctadecylphosphit, Distearyl-pentaerythritdiphosphit, Tris-(2,4-di-tert.butylphenyl)-phosphit, Diisodecylpentaerythrit-diphosphit, Di-(2,4-di-tert.butylphenyl)-pentaerythritdiphosphit, Tristearyl-sorbit-triphosphit, Tetrakis-(2,4-di-tert.butylphenyl)-4,4'-biphenylen-diphosphonit.

5. Peroxidzerstörende Verbindungen, wie z. B. Ester der β-Thio-dipropionsäure, beispielsweise der Lauryl-, Stearyl-, Myristyl- oder Tridecylester, Mercaptobenzimidazol, das Zinksalz des 2-Mercaptobenzimidazols, Zink-dibutyl-dithiocarbamat, Dioctadecyldisulfid, Pentaerythrit-tetrakis-(β-dodecylmercapto)-propionat.

6. Polyamidstabilisatoren, wie z. B. Kupfersalze in Kombination mit Jodiden und/oder Phosphorverbindungen und Salze des zweiwertigen Mangans.

7. Basische Co-Stabilisatoren, wie z. B. Melanin, Polyvinylpyrrolidon, Dicyandiamid, Triallylcyanurat, Harnstoff-Derivate, Hydrazin-Derivate, Amine, Polyamine, Polyurethane, Alkali- und Erdalkalisalze höherer Fettsäuren, beispielsweise Ca-Stearat, Zn-Stearat, Mg-Stearat, Na-Ricinoleat, K-Palmitat, Antimonbrenzcatechinat oder Zinnbrenzcatechinat.

8. Nukleierungsmittel, wie z. B. 4-tert.Butylbenzoesäure, Adipinsäure, Diphenylessigsäure.

9. Füllstoffe und Verstärkungsmittel, wie z. B. Calciumcarbonat, Silikate, Glasfasern, Asbest, Talk, Kaolin, Glimmer, Bariumsulfat, Metalloxide und -hydroxide, Russ, Graphit.

10. Sonstige Zusätze, wie z. B. Weichmacher, Gleitmittel, Emulgatoren, Pigmente, Optische Aufheller, Flammschutzmittel, Antistatika, Treibmittel.

11. Aminische Antioxidantien :

N,N'-Di-isopropyl-p-phenylendiamin
N,N'-Di-sec.-butyl-p-phenylendiamin
N,N'-Bis(1,4-dimethyl-pentyl)-p-phenylendiamin

N,N'-Bis(1-ethyl-3-methyl-pentyl)-p-phenylendiamin
N,N'-Bis(1-methyl-heptyl)-p-phenylendiamin
N,N'-Dicyclohexyl-p-phenylendiamin
N,N'-Diphenyl-p-phenylendiamin
N,N'-Di-(naphthyl-2-)-p-phenylendiamin
N-Isopropyl-N'-phenyl-p-phenylendiamin
N-(1,3-Dimethyl-butyl)-N'-phenyl-p-phenylendiamin
N-(1-Methyl-heptyl)-N'-phenyl-p-phenylendiamin
N-Cyclohexyl-N'-phenyl-p-phenylendiamin
4-(p-Toluol-sulfonamido)-diphenylamin
N,N'-Dimethyl-N,N'-di-sec.-butyl-p-phenylendiamin
Diphenylamin
4-Isopropoxy-diphenylamin
N-Phenyl-1-naphthylamin
N-Phenyl-2-naphthylamin
octyliertes Diphenylamin
4-n-Butylaminophenol
4-Butyrylamino-phenol
4-Nonanoylamino-phenol
4-Dodecanoylamino-phenol
4-Octadecanoylamino-phenol
Di-(4-methoxy-phenyl)-amin
2,6-Di-tert.-butyl-4-dimethylamino-methyl-phenol
2,4'-Diamino-diphenylmethan
4,4'-Diamino-diphenylmethan
N,N,N,N'-Tetramethyl-4,4'-diamino-diphenylmethan
1,2-Di-(phenylamino)-ethan
1,2-Di-[(2-methyl-phenyl)-amino]-ethan
1,3-Di-(phenylamino)-propan
(o-Tolyl)-biguanid
Di-[4-(1',3'-dimethyl-butyl)-phenyl]amin.

12. Metallpassivatoren :

für Kupfer, z. B. :
Benztriazol, Tetrahydrobenztriazol, 2-Mercaptobenzthiazol, 2,5-Dimercaptothiadiazol, Salicylidenpropylendiamin, Salze von Salicylaminoguanidin.

13. Rost-Inhibitoren :

a) Organische Säuren, ihre Ester, Metallsalze und Anhydride, z. B. :
N-Oleoyl-sarcosin, Sorbitan-mono-oleat, Blei-naphthenat, Dodecenylbernsteinsäure-anhydrid, Alkenylbernsteinsäure-Halbester, 4-Nonylphenoxy-essigsäure.

b) Stickstoffhaltige Verbindungen, z. B. :
I. Primäre, sekundäre oder tertiäre aliphatische oder cycloaliphatische Amine und Amin-Salze von organischen und anorganischen Säuren, z. B. öllösliche Alkylammoniumcarboxylate.

II. Heterocyclische Verbindungen z. B. :
Substituierte Imidazoline und Oxazoline.

c) Phosphorhaltige Verbindungen, z. B. :
Aminsalze von Phosphorsäurepartialestern.

d) Schwefelhaltige Verbindungen, z. B. :
Barium-dinonylnaphthalin-sulfonate, Calciumpetroleum-sulfonate.

14. Viskositätsindex-Verbesserer :

Polymethacrylate, Vinylpyrrolidon/Methacrylat-Copolymere, Polybutene, Olefin-Copolymere, Styrol/Acrylat-Copolymere.

15. Stockpunktniedriger :

Polymethacrylat, alkylierte Naphthalinderivate.

16. Dispergiermittel/Tenside :

Polybutenylbernsteinsäure-imide, Polybutenylphosphonsäurederivate, basische Magnesium-, Calcium-, und Bariumsulfonate und -phenolate.

17. Verschleissschutz-Additive :

Schwefel und/oder Phosphor und/oder Halogen enthaltende Verbindungen, wie geschwefelte pflanzliche Oele, Zinkdialkyldithiophosphate, Tritolyl-phosphat, chlorierte Paraffine, Alkyl- und Aryldisulfide.

Herstellungsbeispiele

Beispiel 1 : 10,4 g 2-tert.Butylphenol, 20,5 g 2-Ethylhexylmerkaptan, 10 ml DMF, 0,7 ml Dibutylamin und 8,1 g Paraformaldehyd werden unter Stickstoff während 25 Std. auf 120 °C erhitzt. Nach Abkühlen der Lösung auf Raumtemperatur wird mit Toluol verdünnt und wie üblich mit 2 N-Salzsäure und Wasser neutralgewaschen. Konzentration des Rückstands am Rotavap liefert in guter Ausbeute 2,4-Bis-(2-ethylhexylthiomethyl)-6-tert.butyl-phenol, welches durch Säulen-chromatographie an Kieselgel weiter gereinigt wird ; farbloses Oel, Ber. S 13,74 %, Gef. S 13,56 % (charakteristische Daten in Tabelle 2).

Beispiel 2 : 60,1 g 2-tert.-Butylphenol, 170 g Dodecylmerkaptan, 60 ml DMF, 3,1 g Dibutylamin und 24,2 g Paraformaldehyd werden unter Stickstoff während 24 Stunden auf 130 °C erhitzt. Aufarbeitung wie unter Beispiel 1 liefert 2,4-Bis-dodecylthiomethyl-6-tert.-butylphenol ; farbloses Oel, Ber. S 11,07 %, Gef. S 11,03 % (charakteristische Daten in Tabelle 2).

Beispiel 3 : Die unter Beispiel 2 beschriebene Verbindung wird auch erhalten, wenn 26,4 g Bis-2,4-dimethylaminomethyl-6-tert.-butylphenol, 40,5 g Dodecylmerkaptan und 200 ml DMF während 20 Stunden auf 140 °C erhitzt werden. Ber. S 11,07 %, Gef. S 11,30 % (charakteristische Daten in Tabelle 2).

Beispiel 4 : Verfährt man wie unter Beispiel 3 beschrieben, ausser dass man 43,7 g 2-Merkaptopropionsäure-2-ethylhexylester statt Dodecylmerkaptan einsetzt, erhält man 2,4-Bis-[2-(2-ethylhexyloxycarbonyl) ethylthiomethyl]-6-tert.-butylphenol ; farbloses Oel, Ber. S 10,50 %, Gef. S 10,52 % (charakteristische Daten in Tabelle 2).

In Analogie zum Verfahren des Beispiels 1 bzw. 3 werden die in der folgenden Tabelle 1 aufgelisteten Verbindungen erhalten

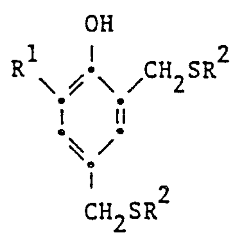

(Siehe Tabelle 1 Seite 15 ff.)

Tabelle 1

| Nr. | $R^1$ | $R^2$ | Verfahren [1] | Charakterisierung | Analyse (S) Ber. (%) | Gef. (%) |
|---|---|---|---|---|---|---|
| 5[2] | $-C(CH_3)_3$ | $-(CH_2)_7-CH_3$ | b | Oel | 13,73 | 13,42 |
| 6[2] | $-C_6H_5$ | $-CH_2-COO-CH_2-CH-(CH_2)_3-CH_3$<br>\|<br>$C_2H_5$ | a | Oel | 10,64 | 10,86 |
| 7[2] | $-C(CH_3)_3$ | $-CH_2-COO-CH_2-CH-(CH_2)_3-CH_3$<br>\|<br>$C_2H_5$ | b | Oel | 11,00 | 10,86 |
| 8[2] | $-C(CH_3)_3$ | 1:1 Gemisch aus<br>$-C(CH_3)_2-CH_2-C(CH_3)_2-CH_2-C(CH_3)_3$<br>und $-C(CH_3)(CH_2-C(CH_3)_3)_2$ | b | gelbliches, klares Oel | 11,07 | 11,03 |

[1] a : Verfahren nach Beispiel 1
b : Verfahren nach Beispiel 3
[2] charakterische Daten in Tabelle 2

Tabelle 1 (Fortsetzung)

| Nr. | $R^1$ | $R^2$ | Verfahren[1)] | Charakterisierung | Analyse (S) Ber. % | Gef. % |
|---|---|---|---|---|---|---|
| 9[2)] | $-C(CH_3)_3$ | $t-C_8H_{17}-$ | a | Smp. 61° | 13,74 | 13,63 |
| 10[2)] | $-C(CH_3)_3$ | $t-C_9H_{19}-$ | b | Smp. 50° | 12,96 | 13,57 |
| 11[2)] | $-C(CH_3)_3$ | $-CH_2CH_2OH$ | b | Oel | 19,40 | 19,20 |
| 12[2)] | $-C(CH_3)_3$ | $-CH_2-CH(OH)CH_2OH$ | b | Oel | 16,42 | 16,05 |
| 13[2)] | $-C(CH_3)_3$ | 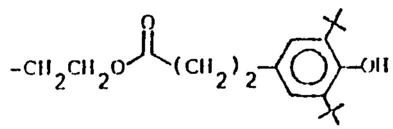 | a | Harz | 7,53 | 7,33 |

1) a : Verfahren nach Beispiel 1
b : Verfahren nach Beispiel 3
2) charakterische Daten in Tabelle 2

Typische Signale im 1H-NMR-Spektrum der beschriebenen Verbindungen

Die Methylenprotonen der Gruppierungen Aryl-$CH_2$-S absorbieren im $^1$H-NMR-Spektrum ($CDCl_3$) als 2 Singulette. Die chemischen Verschiebungen sind in Tabelle 2 zusammengefasst (Standard TMS).

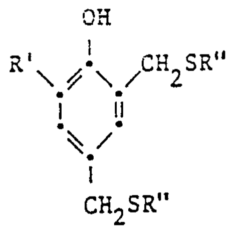

(Siehe Tabelle 2 Seite 18 ff.)

Tabelle 2

| Bsp. Nr. | R' | R'' | Signal Aryl-$\underline{CH_2}$-S- ortho-$CH_2$ (ppm) | para-$CH_2$ (ppm) | Gerätetyp |
|---|---|---|---|---|---|
| 1 | $-C(CH_3)_3$ | $-CH_2-CH(C_2H_5)-(CH_2)_3-CH_3$ | 3,79 | 3,60 | b |
| 2/3 | $-C(CH_3)_3$ | $-(CH_2)_{11}-CH_3$ | 3,76 | 3,60 | b |
| 4 | $-C(CH_3)_3$ | $-(CH_2)_2-COO-CH_2-CH(C_2H_5)-(CH_2)_3-CH_3$ | 3,77 | 3,60 | a |
| 5 | $-C(CH_3)_3$ | $-(CH_2)_7-CH_3$ | 3,78 | 3,62 | c |
| 6 | $-C(CH_3)_3$ | $-CH_2-COO-CH_2-CH(C_2H_5)-(CH_2)_3-CH_3$ | 3,86 | 3,73 | b |
| 7 | $-C_6H_5$ | $-CH_2-COO-CH_2-CH(C_2H_5)-(CH_2)_3-CH_3$ | 3,94 | 3,80 | b |
| 8 | $-C(CH_3)_3$ | Gemisch aus $-C(CH_3)_2-CH_2-C(CH_3)_2-CH_2-C(CH_3)_3$ $-C(CH_3)(CH_2-C(CH_3)_3)_2$ | 3,77 und 3,70 | 3,61 und 3,54 | b |

a: 60 MHz
b: 250 MHz
c: 100 MHz

Tabelle 2 (Fortsetzung)

| Bsp. Nr. | R' | R" | Signal Aryl-$\underline{CH_2}$-S ortho-$CH_2$ (ppm) | para-$CH_2$ (ppm) | Gerätetyp |
|---|---|---|---|---|---|
| 9 | $-C(CH_3)_3$ | $t-C_8H_{17}-$ | 3,80 | 3,65 | c |
| 10 | $-C(CH_3)_3$ | $t-C_9H_{19}-$ | 3,84 | 3,66 | c |
| 11 | $-C(CH_3)_3$ | $-CH_2CH_2OH$ | 3,85 | 3,66 | c |
| 12 | $-C(CH_3)_3$ | $-CH_2CH(OH)CH_2OH$ | 3,81 | 3,64 | c |
| 13 | $-C(CH_3)_3$ | $-CH_2CH_2O\overset{O}{\overset{\|}{C}}(CH_2)_2-$ 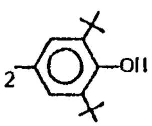 | 3,82 | 3,67 | c |

a: 60 MHz
b: 250 MHz
c: 100 MHz

Anwendungsbeispiel

TOST-TEST, Oxidation Charakteristika von Mineralöl Mobil 150 SS4 (ASTM D934/DIN 51587/IP 157)

Das zu testende Oel wird in Gegenwart von Wasser, Sauerstoff, einem Eisen-Kupferkatalysator und dem jeweiligen Stabilisator während 500 Stunden auf 95 °C erwärmt. Danach wird der Säurewert (in mg KOH-Verbrauch pro g Testöl) sowie der Schlamm (in mg Rückstand pro Ansatz) bestimmt. Die Resultate sind in Tabelle 3 zusammengestellt. Die Konzentration des Stabilisators beträgt 0,25 Gew.-% bezogen auf das Oel.

Tabelle 3

| Stabilisator | Säurewert (mg KOH/g) | Schlamm (mg) |
|---|---|---|
| Beispiel 2 | 0,21 | 3,9 |
| Beispiel 5 | 0,22 | 1,0 |

**Patentansprüche**

1. Verbindungen der Formel I

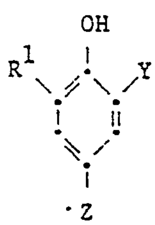

(I)

worin $R^1$ $C_2$-$C_{20}$-Alkyl, $C_3$-$C_{20}$-Alkenylmethyl, $C_3$-$C_{20}$-Alkinylmethyl, $C_5$-$C_{12}$-Cycloalkyl, Phenyl, 1- oder 2-Naphthyl, $C_7$-$C_{14}$-Alkaryl oder $C_7$-$C_{14}$-Aralkyl bedeutet, Y $—CH_2—S—R^2$ ist, Z $—CH_2—S—R^3$ bedeutet oder worin Y und/oder Z ein Rest

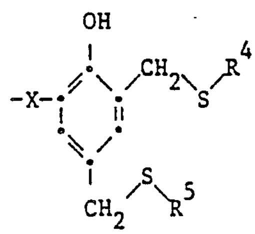

ist, worin $R^2$, $R^3$, $R^4$ und $R^5$ unabhängig voneinander $C_1$-$C_{20}$-Alkyl, durch einen Phenylrest und/oder eine oder zwei Hydroxygruppen substituiertes $C_2$-$C_{20}$-Alkyl, $C_3$-$C_{20}$-Alkenylmethyl, $C_3$-$C_{20}$-Alkinylmethyl, $C_5$-$C_{12}$-Cycloalkyl, Phenyl, 1- oder 2-Naphthyl, $C_7$-$C_{14}$-Alkaryl, $C_7$-$C_{14}$-Aralkyl oder durch Hydroxy in 2-Stellung substituiertes $C_5$-$C_7$-Cycloalkyl oder 1,3-Benzthiazol-2-yl sind, oder Reste der Formeln $—C(R^6R^7)—(CHR^8)_m—W$, $—(CH_2)_2—OCO—R^{15}$ oder

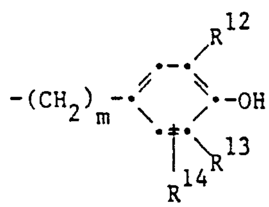

bedeuten, worin m 0, 1 oder 2 ist, $R^6$, $R^7$ und $R^8$ unabhängig voneinander Wasserstoff oder $C_1$-$C_6$-Alkyl sind, W ein Rest $—COR^9$, $—CN$, $—COOR^{10}$ oder $—CON(R^{10}R^{11})$ ist, wobei $R^9$ Wasserstoff, $C_1$-$C_{20}$-Alkyl, Phenyl, 1- oder 2-Naphthyl, $C_5$-$C_{12}$-Cycloalkyl, $C_7$-$C_{14}$-Aralkyl oder $C_7$-$C_{14}$-Alkaryl ist, und worin $R^{10}$ und $R^{11}$ unabhängig voneinander die Bedeutungen von $R^9$ besitzen, oder zusätzlich durch eine Hydroxy-

oder Cyangruppe substituiertes $C_2$-$C_{20}$-Alkyl sind, oder durch ein bis fünf —O—, —S—, —$N(CH_3)$— oder —$N(C_2H_5)$— unterbrochenes gegebenenfalls mit einer Hydroxygruppe substituiertes $C_3$-$C_{20}$-Alkyl bedeuten, wobei gegebenenfalls mehrfach auftretende Heteroatome durch mindestens eine Methylengruppe getrennt sein müssen, worin $R^{10}$ oder $R^{11}$ $C_2$-$C_{12}$-Alkenyl, $C_2$-$C_{12}$-Alkinyl, durch ein oder zwei —$NO_2$, —Cl, —Br, —$OCH_3$ oder —$COOR^{18}$ substituiertes Phenyl oder eine Gruppe

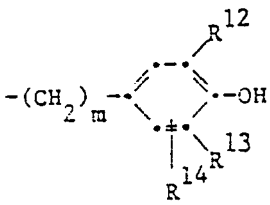

bedeuten, oder worin $R^{10}$ und $R^{11}$ zusammen mit dem Stickstoffatom einen fünf-, sechs- oder siebengliedrigen heterozyklischen Ring bilden, der gegebenenfalls noch ein weiteres Heteroatom enthalten kann, worin $R^{12}$, $R^{13}$ und $R^{14}$ unabhängig voneinander Wasserstoff, $C_1$-$C_{20}$-Alkyl, Cyclohexyl oder Phenyl sind, worin $R^{15}$ $C_1$-$C_{20}$-Alkyl, $C_5$-$C_{12}$-Cycloalkyl, $C_7$-$C_{14}$-Aralkyl, $C_7$-$C_{14}$-Alkaryl, Phenyl, 1- oder 2-Naphthyl oder ein Rest

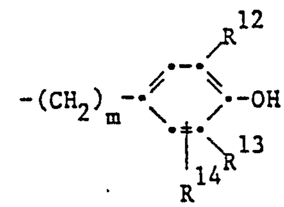

bedeutet, worin X —$C(R^{16}R^{17})$—, —S— oder —S—S— ist, wobei $R^{16}$ und $R^{17}$ unabhängig voneinander Wasserstoff, $C_1$-$C_6$-Alkyl, Cyclohexyl oder Phenyl bedeuten, und worin schliesslich $R^{18}$ $C_1$-$C_6$-Alkyl, Cyclohexyl, Phenyl, Benzyl oder Tolyl ist.

2. Verbindungen der Formel I gemäss Anspruch 1, worin $R^1$ verzweigtes $C_3$-$C_{12}$-Alkyl bedeutet.

3. Verbindungen der Formel I gemäss Anspruch 1, worin $R^1$ tert.-Butyl ist.

4. Verbindungen der Formel I gemäss Anspruch 1, worin Y —$CH_2$—S—$R^2$ und Z —$CH_2S$—$R^3$ bedeuten.

5. Verbindungen der Formel I gemäss Anspruch 1, worin $R^1$ verzweigtes $C_3$-$C_{12}$-Alkyl, $C_5$-$C_9$-Cycloalkyl, Phenyl oder $C_7$-$C_9$-Aralkyl ist, worin $R^2$ und $R^3$ unabhängig voneinander $C_4$-$C_{14}$-Alkyl, durch ein Phenyl und/oder eine oder zwei Hydroxygruppen substituiertes $C_2$-$C_{14}$-Alkyl, Allyl, Propargyl, $C_5$-$C_9$-Cycloalkyl, 2-Hydroxycyclohexyl, Phenyl, 1,3-Benzthiazol-2-yl oder $C_7$-$C_9$-Aralkyl sind, oder ein Rest —$C(R^6R^7)$—$(CHR^8)_m$—W, —$(CH_2)_2$—OCO—$R^{15}$ oder

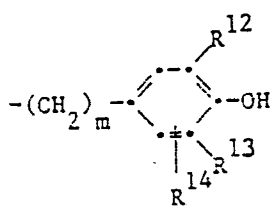

sind, worin m 0, 1 oder 2 bedeutet, und worin $R^6$, $R^7$ und $R^8$ unabhängig voneinander Wasserstoff oder Methyl sind, W —$COOR^{10}$ oder —$CON(R^{10}R^{11})$ bedeutet, wobei $R^{10}$ und $R^{11}$ unabhängig voneinander Wasserstoff, $C_1$-$C_{12}$-Alkyl, Phenyl, $C_5$-$C_9$-Cycloalkyl, $C_7$-$C_9$-Aralkyl, 2-Hydroxyethyl, 2-Cyanethyl oder durch ein bis drei —O— unterbrochenes gegebenenfalls durch eine Hydroxygruppe substituiertes $C_3$-$C_{12}$-Alkyl sind, wobei gegebenenfalls mehrfach auftretende Sauerstoffatome durch mindestens eine Methylengruppe getrennt sein müssen, worin $R^{10}$ und $R^{11}$ Allyl, Propargyl, durch ein —Cl, —$COOCH_3$ oder —$OCH_3$ substituiertes Phenyl oder einen Rest

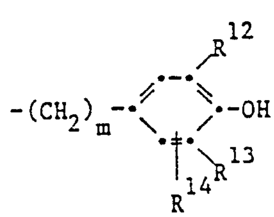

bedeuten, oder worin $R^{10}$ und $R^{11}$ zusammen mit dem gemeinsamen Stickstoffatom einen Pyrrol-, Piperidin-, Pyrrolidin-, Hexamethylenimin- oder Morpholinring bilden, worin $R^{12}$, $R^{13}$ und $R^{14}$ unabhängig voneinander Wasserstoff oder $C_1$-$C_{12}$-Alkyl sind, worin $R^{15}$ $C_1$-$C_{12}$-Alkyl, $C_5$-$C_9$-Cycloalkyl, $C_7$-$C_9$-Aralkyl, Phenyl oder ein Rest

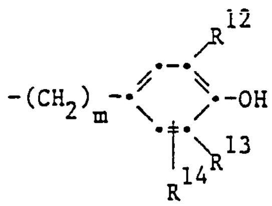

ist.

6. Verbindungen der Formel I gemäss Anspruch 1, worin $R^1$ tert.-Butyl, Cyclohexyl, Phenyl oder Benzyl bedeutet, und worin $R^2$ und $R^3$ unabhängig voneinander $C_4$-$C_{14}$-Alkyl, 2-Hydroxyethyl, 2,3-Dihydroxypropyl, 1-Phenyl-2-hydroxyethyl, Cyclohexyl, Phenyl oder Benzyl darstellen.

7. Verbindungen der Formel I gemäss Anspruch 1, worin $R^1$ tert.-Butyl, Cyclohexyl, Phenyl oder Benzyl bedeutet, worin $R^2$ und $R^3$ Reste der Formel $-(CH_2)_{m+1}-W$ sind, wobei W $-COOR^{10}$ oder $-CON(R^{10}R^{11})$ bedeutet und wobei $R^{10}$ und $R^{11}$ unabhängig voneinander Wasserstoff, $C_1$-$C_{12}$-Alkyl, Phenyl, Cyclohexyl, Benzyl, 2-Hydroxyethyl, 2-Hydroxypropyl, 5-Hydroxy-3-oxapentyl oder 3-Oxabutyl bedeuten, oder ein Rest

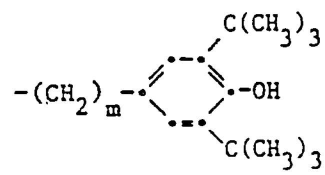

sind oder wobei $R^{10}$ und $R^{11}$ zusammen mit dem gemeinsamen Stickstoffatom einen Piperidin- oder Morpholinring bilden.

8. Verbindungen der Formel I gemäss Anspruch 1, worin $R^1$ tert.-Butyl ist und $R^2$ und $R^3$ die gleichen Reste n-Octyl, t-Octyl, n-Dodecyl oder t-Dodecyl bedeuten.

9. Verbindung der Formel 1 gemäss Anspruch 8, worin $R^2$ und $R^3$ n-Octyl bedeuten.

10. Verbindung der Formel 1 gemäss Anspruch 8, worin $R^2$ und $R^3$ n-Dodecyl bedeuten.

11. Zusammensetzungen enthaltend ein gegen thermischen, oxidativen oder strahlungsinduzierten Abbau empfindliches organisches Material und mindestens eine Verbindung der Formel I gemäss Anspruch 1.

12. Zusammensetzungen gemäss Anspruch 11, worin das organische Material ein Polymeres oder ein Schmierstoff auf Mineralölbasis oder ein synthetischer Schmierstoff ist.

13. Zusammensetzungen gemäss Anspruch 11, enthaltend mindestens eine Verbindung gemäss Anspruch 6, und worin das organische Material ein Schmierstoff auf Mineralölbasis oder ein synthetischer Schmierstoff ist.

14. Zusammensetzungen gemäss Anspruch 11, enthaltend mindestens eine Verbindung gemäss Anspruch 7, und worin das organische Material ein Elastomeres oder ein Schmierstoff auf Mineralölbasis oder ein synthetischer Schmierstoff ist.

15. Verwendung von Verbindungen der Formel I gemäss Anspruch 1 als Stabilisatoren für organisches Material gegen dessen Schädigung durch Einwirkung von Sauerstoff, Wärme, Licht, oder energiereicher Strahlung.

16. Verwendung der Verbindungen der Formel I gemäss Anspruch 15 als Antioxidantien in organischen Polymeren oder in Mineralölen oder in synthetischen Oelen.

## Claims

1. A compound of the formula I

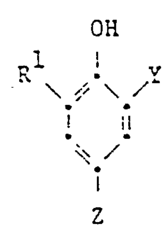

(I)

in which $R^1$ is $C_2$-$C_{20}$ alkyl, $C_3$-$C_{20}$ alkenylmethyl, $C_3$-$C_{20}$ alkinylmethyl, $C_5$-$C_{12}$ cycloalkyl, phenyl, 1- or 2-naphthyl, $C_7$-$C_{14}$ alkaryl or $C_7$-$C_{14}$ aralkyl, Y is —$CH_2$—S—$R^2$ and Z is —$CH_2$—S—$R^3$, or in which Y and/or Z is a radical

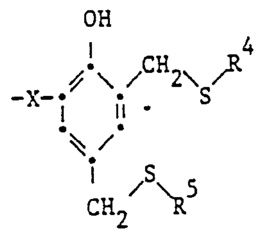

in which $R^2$, $R^3$, $R^4$ and $R^5$ independently of one another are $C_1$-$C_{20}$ alkyl, $C_2$-$C_{20}$ alkyl which is substituted by a phenyl radical and/or one or two hydroxyl groups, $C_3$-$C_{20}$-alkenylmethyl, $C_3$-$C_{20}$ alkinylmethyl, $C_5$-$C_{12}$ cycloalkyl, phenyl, 1- or 2-naphthyl, $C_7$-$C_{14}$ alkaryl, $C_7$-$C_{14}$ aralkyl, $C_5$-$C_7$ cycloalkyl which is substituted by hydroxyl in the 2-position, or 1,3-benzothiazol-2-yl, or radicals of the formulae —C($R^6R^7$)—($CHR^8$)$_m$—W, —$(CH_2)_2$—OCO—$R^{15}$ or

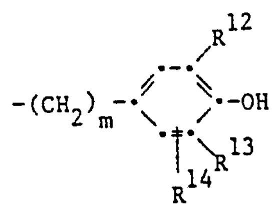

in which m is 0, 1 or 2, $R^6$, $R^7$ and $R^8$ independently of one another are hydrogen or $C_1$-$C_6$ alkyl, W is a radical —$COR^9$, —CN, —$COOR^{10}$ or —CON($R^{10}R^{11}$), in which $R^9$ is hydrogen, $C_1$-$C_{20}$ alkyl, phenyl, 1- or 2-naphthyl, $C_5$-$C_{12}$-cycloalkyl, $C_7$-$C_{14}$ aralkyl or $C_7$-$C_{14}$ alkaryl and in which $R^{10}$ and $R^{11}$ independently of one another have the meanings of $R^9$, or are additionally $C_2$-$C_{20}$ alkyl which is substituted by a hydroxyl or cyano group, or are $C_3$-$C_{20}$ alkyl which is interrupted by one to five —O—, —S—, —N($CH_3$)— or —N($C_2H_5$)— and is unsubstituted or substituted by a hydroxyl group, it being necessary for the several heteroatoms which may occur to be separated by at least one methylene group, in which $R^{10}$ or $R^{11}$ is $C_2$-$C_{12}$ alkenyl, $C_2$-$C_{12}$ alkinyl, phenyl which is substituted by one or two —$NO_2$, —Cl, —Br, —$OCH_3$ or —$COOR^{18}$, or a group

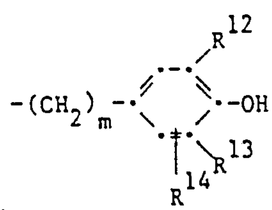

or in which $R^{10}$ and $R^{11}$, together with the nitrogen atom, form a five-, six- or seven-membered heterocyclic ring, which may also contain a further heteroatom, in which $R^{12}$, $R^{13}$ and $R^{14}$ independently of one another are hydrogen, $C_1$-$C_{20}$-alkyl, cyclohexyl or phenyl, in which $R^{15}$ is $C_1$-$C_{20}$ alkyl, $C_5$-$C_{12}$ cycloalkyl, $C_7$-$C_{14}$ aralkyl, $C_7$-$C_{14}$ alkaryl, phenyl, 1- or 2-naphthyl or a radical

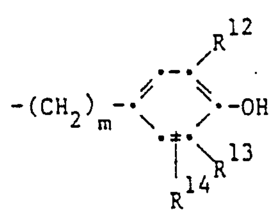

in which X is —C($R^{16}R^{17}$)—, —S— or —S—S—, in which $R^{16}$ and $R^{17}$ independently of one another are hydrogen, $C_1$-$C_6$ alkyl, cyclohexyl or phenyl, and in which, finally, $R^{18}$ is $C_1$-$C_6$-alkyl, cyclohexyl, phenyl, benzyl or tolyl.

2. A compound of the formula I according to claim 1, in which $R^1$ is branched $C_3$-$C_{12}$-alkyl.

3. A compound of the formula I according to claim 1, in which $R^1$ is tert-butyl.

4. A compound of the formula I according to claim 1, in which Y is —$CH_2$—S—$R^2$ and Z is —$CH_2$S—$R^3$.

5. A compound of the formula I according to claim 1, in which $R^1$ is branched $C_3$-$C_{12}$ alkyl, $C_5$-$C_9$ cycloalkyl, phenyl or $C_7$-$C_9$ aralkyl, in which $R^2$ and $R^3$ independently of one another are $C_4$-$C_{14}$ alkyl, $C_2$-$C_{14}$ alkyl which is substituted by a phenyl and/or one or two hydroxyl groups, allyl, propargyl, $C_5$-$C_9$ cycloalkyl, 2-hydroxycyclohexyl, phenyl, 1,3-benzothiazol-2-yl or $C_7$-$C_9$ aralkyl, or are a radical $-C(R^6R^7)-(CHR^8)_m-W$, $-(CH_2)_2-OCO-R^{15}$ or

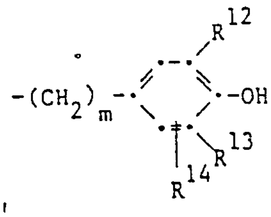

in which m is 0, 1 or 2, and in which $R^6$, $R^7$ and $R^8$ independently of one another are hydrogen or methyl, W is $-COOR^{10}$ or $-CON(R^{10}R^{11})$, in which $R^{10}$ and $R^{11}$ independently of one another are hydrogen, $C_1$-$C_{12}$ alkyl, phenyl, $C_5$-$C_9$-cycloalkyl, $C_7$-$C_9$ aralkyl, 2-hydroxyethyl, 2-cyanoethyl, or $C_3$-$C_{12}$ alkyl which is interrupted by one to three $-O-$ and is unsubstituted or substituted by a hydroxyl group, it being necessary for several oxygen atoms which may occur to be separated by at least one methylene group, in which $R^{10}$ and $R^{11}$ are allyl, propargyl, phenyl which is substituted by a $-Cl$, $-COOCH_3$ or $-OCH_3$, or a radical

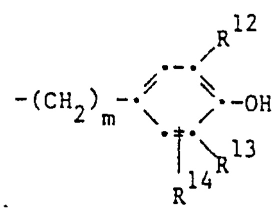

or in which $R^{10}$ and $R^{11}$, together with the common nitrogen atom, form a pyrrole, piperidine, pyrrolidine, hexamethyleneimine or morpholine ring, in which $R^{12}$, $R^{13}$ and $R^{14}$ independently of one another are hydrogen or $C_1$-$C_{12}$ alkyl, and in which $R^{15}$ is $C_1$-$C_{12}$ alkyl, $C_5$-$C_9$ cycloalkyl, $C_7$-$C_9$ aralkyl, phenyl or a radical

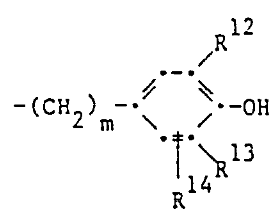

6. A compound of the formula I according to claim 1, in which $R^1$ is tert-butyl, cyclohexyl, phenyl or benzyl, and in which $R^2$ and $R^3$ independently of one another are $C_4$-$C_{14}$-alkyl, 2-hydroxyethyl, 2,3-dihydroxypropyl, 1-phenyl-2-hydroxyethyl, cyclohexyl, phenyl or benzyl.

7. A compound of the formula I according to claim 1, in which $R^1$ is tert-butyl, cyclohexyl, phenyl or benzyl, in which $R^2$ and $R^3$ are radicals of the formula $-(CH_2)_{m+1}-W$, in which W is $-COOR^{10}$ or $-CON(R^{10}R^{11})$, and in which $R^{10}$ and $R^{11}$ independently of one another denote hydrogen, $C_1$-$C_{12}$-alkyl, phenyl, cyclohexyl, benzyl, 2-hydroxyethyl, 2-hydroxypropyl, 5-hydroxy-3-oxapentyl or 3-oxabutyl, or are a radical

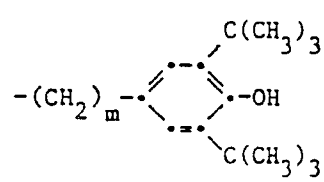

or in which $R^{10}$ and $R^{11}$, together with the common nitrogen atom form a piperidine or morpholine ring.

8. A compound of the formula I according to claim 1, in which $R^1$ is tert-butyl and $R^2$ and $R^3$ are the same n-octyl, t-octyl, n-dodecyl or t-dodecyl radicals.

9. The compound of the formula I according to claim 8, in which $R^2$ and $R^3$ are n-octyl.

10. The compound of the formula I according to claim 8, in which $R^2$ and $R^3$ are n-dodecyl.

11. A composition containing an organic material which is sensitive towards thermal, oxidative or radiation-induced degradation and at least one compound of the formula I according to claim 1.

12. A composition according to claim 11, in which the organic material is a polymer, a lubricant based on a mineral oil or a synthetic lubricant.

13. A composition according to claim 11, containing at least one compound according to claim 6, and in which the organic material is a lubricant based on a mineral oil or a synthetic lubricant.

14. A composition according to claim 11, containing at least one compound according to claim 7, and in which the organic material is an elastomer, a lubricant based on a mineral oil or a synthetic lubricant.

15. The use of a compound of the formula I according to claim 1 as a stabiliser for stabilising organic material against damage caused by the action of oxygen, heat, light or high-energy radiation.

16. The use of a compound of the formula I according to claim 15 as an antioxidant in an organic polymer, a mineral oil or a synthetic oil.

**Revendications**

1. Composés de formule I :

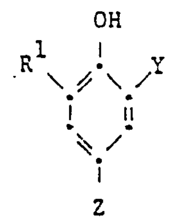

(I)

dans laquelle $R^1$ désigne un $C_2$-$C_{20}$-alkyle, un $C_3$-$C_{20}$-alcénylméthyle, un $C_3$-$C_{20}$-alcynylméthyle, un $C_5$-$C_{12}$-cycloalkyle, un phényle, un 1- ou 2-naphtyle, un $C_7$-$C_{14}$-alcaryle ou un $C_7$-$C_{14}$-aralkyle, Y un groupe $—CH_2—S—R^2$ et Z un groupe $—CH_2—S—R^3$, ou bien Y et/ou Z sont des radicaux :

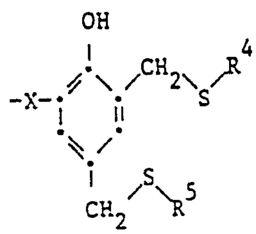

$R^2$, $R^3$, $R^4$ et $R^5$ représentant chacun, indépendamment les uns des autres, un $C_1$-$C_{20}$-alkyle, un $C_2$-$C_{20}$-alkyle portant un phényle et/ou un ou deux groupes hydroxy, un $C_3$-$C_{20}$-alcénylméthyle, un $C_3$-$C_{20}$-alcynylméthyle, un $C_5$-$C_{12}$-cycloalkyle, un phényle, un 1- ou 2-naphtyle, un $C_7$-$C_{14}$-alcaryle, un $C_7$-$C_{14}$-aralkyle, un $C_5$-$C_7$-cycloalkyle avec un hydroxyle à la position 2, un radical 1,3-benzothiazol-2-yle ou encore des radicaux $—C(R^6R^7)—(CHR^8)_m—W$, $—(CH_2)_2—OCO—R^{15}$ ou

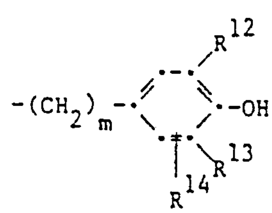

radicaux dans lesquels m est le nombre 0, 1 ou 2, $R^6$, $R^7$ et $R^8$ désignent chacun, indépendamment les uns des autres, l'hydrogène ou un $C_1$-$C_6$-alkyle, W est un radical $—COR^9$, $—CN$, $—COOR^{10}$ ou $—CON(R^{10}R^{11})$, $R^9$ étant l'hydrogène, un $C_1$-$C_{20}$-alkyle, un phényle, un 1- ou -2-naphtyle, un $C_5$-$C_{12}$-cycloalkyle, un $C_7$-$C_{14}$-aralkyle ou un $C_7$-$C_{14}$-alcaryle et $R^{10}$ et $R^{11}$, indépendamment l'un de l'autre, ayant les mêmes significations que $R^9$ ou bien peuvent être aussi des $C_2$-$C_{20}$-alkyles avec un hydroxyle ou un groupe cyano ou encore des $C_3$-$C_{20}$-alkyles avec un hydroxyle et pouvant être éventuellement interrompus par un à cinq atomes d'oxygène ou de soufre ou groupes $—N(CH_3)—$ ou $—N(C_2H_5)—$, et s'il y a plusieurs hétéroatomes ils doivent être séparés par au moins un groupe méthylène, $R^{10}$ ou $R^{11}$ pouvant être aussi un $C_2$-$C_{12}$-alcényle, un $C_2$-$C_{12}$-alcynyle, un phényle avec un ou deux substituants, à savoir $—NO_2$, $—Cl$, $—Br$, $—OCH_3$ ou $—COOR^{18}$ ou un groupe :

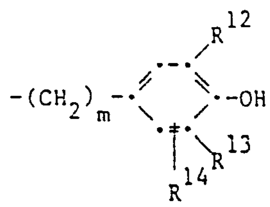

ou encore $R^{10}$ et $R^{11}$ peuvent former ensemble avec l'atome d'azote un hétérocycle pentagonal, hexagonal ou heptagonal pouvant éventuellement avoir un autre hétéroatome, $R^{12}$, $R^{13}$ et $R^{14}$ désignent chacun, indépendamment les uns des autres, l'hydrogène, un $C_1$-$C_{20}$-alkyle, un cyclohexyle ou un phényle, $R^{15}$ est un $C_1$-$C_{20}$-alkyle, un $C_5$-$C_{12}$-cycloalkyle, un $C_7$-$C_{14}$-aralkyle, un $C_7$-$C_{14}$-alcaryle, un phényle un 1- ou 2-naphtyle ou un radical :

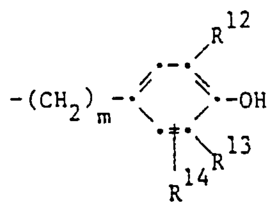

X désignant —$C(R^{16}R^{17})$—, —S— ou —S—S—, $R^{16}$ et $R^{17}$ étant chacun, indépendamment l'un de l'autre, un $C_1$-$C_6$-alkyle, un cyclohexyle ou un phényle, et enfin $R^{18}$ représentant un $C_1$-$C_6$-alkyle ou un groupe cyclohexyle, phényle, benzyle ou tolyle.

2. Composés de formule I selon la revendication 1 dans lesquels $R^1$ est un alkyle en $C_3$-$C_{12}$ à chaîne ramifiée.

3. Composés de formule I selon la revendication 1 dans lesquels $R^1$ est le groupe butyle tertiaire.

4. Composés de formule I selon la revendication 1 dans lesquels Y est un groupe —$CH_2$—S—$R^2$ et Z un groupe —$CH_2S$—$R^3$.

5. Composés de formule I selon la revendication 1 dans lesquels $R^1$ est un $C_3$-$C_{12}$-alkyle à chaîne ramifiée, un $C_5$-$C_9$-cycloalkyle, un phényle ou un $C_7$-$C_9$-aralkyle, $R^2$ et $R^3$ étant chacun un $C_4$-$C_{14}$-alkyle, un $C_{12}$-$C_{14}$-alkyle avec un phényle et/ou un ou deux hydroxyles, un groupe allyle ou propargyle, un $C_5$-$C_9$-cycloalkyle, un 2-hydroxycyclohexyle, un phényle, un 1,3-benzothiazol-2-yle ou un $C_7$-$C_9$-aralkyle ou encore un radical —$C(R^6R^7)$—$(CHR^8)_m$—W, —$(CH_2)_2$—OCO—$R^{15}$ ou

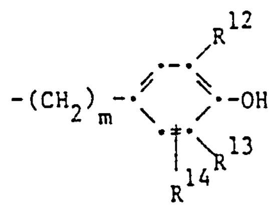

m étant le nombre 0, 1 ou 2, $R^6$, $R^7$ et $R^8$ chacun l'hydrogène ou le groupe méthyle, W un groupe —$COOR^{10}$ ou —$CON(R^{10}R^{11})$, $R^{10}$ et $R^{11}$ étant chacun l'hydrogène, un $C_1$-$C_{12}$-alkyle, un phényle, un $C_5$-$C_9$-cycloalkyle, un $C_7$-$C_9$-aralkyle, un 2-hydroxyéthyle, un 2-cyanoéthyle ou un $C_3$-$C_{12}$-alkyle avec éventuellement un hydroxyle, interrompu par un à trois atomes d'oxygène, et s'il y a plusieurs atomes d'oxygène, ceux-ci doivent être séparés par au moins un groupe méthylène, $R^{10}$ et $R^{11}$ pouvant être aussi chacun un groupe allyle, propargyle, un groupe phényle portant un atome de chlore ou un groupe —$COOCH_3$ ou —$OCH_3$ ou encore un radical :

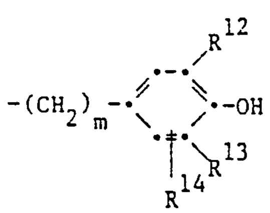

ou encore $R^{10}$ et $R^{11}$ pouvant former ensemble et avec l'atome d'azote un cycle de pyrrole, de pipéridine, de pyrrolidine, d'hexaméthylène-imide ou de morpholine, $R^{12}$, $R^{13}$ et $R^{14}$ étant chacun l'hydrogène ou un $C_1$-$C_{12}$-alkyle, et $R^{15}$ un $C_1$-$C_{12}$-alkyle, un $C_5$-$C_9$-cycloalkyle, un $C_7$-$C_9$-aralkyle, un phényle ou un radical :

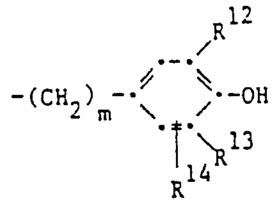

6. Composés de formule I selon la revendication 1 dans lesquels $R^1$ est le groupe tert-butyle, cyclohexyle, phényle ou benzyle et $R^2$ et $R^3$ sont chacun, indépendamment l'un de l'autre, un alkyle en $C_4$-$C_{14}$ ou le groupe 2-hydroxyéthyle, 2,3-dihydroxypropyle, 1-phényl-2-hydroxyéthyle, cyclohexyle, phényle ou benzyle.

7. Composés de formule I selon la revendication 1 dans lesquels $R^1$ est le groupe tert-butyle, cyclohexyle, phényle ou benzyle et $R^2$ et $R^3$ sont des radicaux $-(CH_2)_{m+1}-W$, W étant un groupe $-COOR^{10}$ ou $-CON(R^{10}R^{11})$, $R^{10}$ et $R^{11}$ désignant chacun, indépendamment l'un de l'autre, l'hydrogène, un alkyle en $C_1$-$C_{12}$ ou le groupe phényle, cyclohexyle, benzyle, 2-hydroxyéthyle, 2-hydroxypropyle, 5-hydroxy-3-oxapentyle ou 3-oxabutyle ou encore un radical :

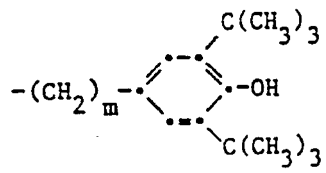

ou bien $R^{10}$ et $R^{11}$ formant ensemble et avec l'atome d'azote un cycle de pipéridine ou de morpholine.

8. Composés de formule I selon la revendication 1 dans lesquels $R^1$ est le groupe tert-butyle et $R^2$ et $R^3$ sont des radicaux identiques, n-octyle, t-octyle, n-dodécyle ou t-dodécyle.

9. Composés de formule I selon la revendication 8 dans lesquels $R^2$ et $R^3$ sont chacun le groupe n-octyle.

10. Composés de formule I selon la revendication 8 dans lesquels $R^2$ et $R^3$ sont chacun le groupe n-dodécyle.

11. Compositions qui comprennent une matière organique sensible aux dégradations sous l'action de la chaleur, de l'oxydation ou d'un rayonnement, avec un ou plusieurs composés de formule I selon la revendication 1.

12. Compositions selon la revendication 11 dans lesquelles la matière organique est une matière polymère, un lubrifiant d'huile minérale ou un lubrifiant synthétique.

13. Compositions selon la revendication 11 qui contiennent un ou plusieurs composés de la revendication 6 et dont la matière organique est un lubrifiant d'huile minérale ou un lubrifiant synthétique.

14. Compositions selon la revendication 11 qui contiennent un ou plusieurs composés de la revendication 7 et dont la matière organique est un élastomère ou bien un lubrifiant d'huile minérale ou un lubrifiant synthétique.

15. Emploi de composés de formule I selon la revendication 1 comme stabilisants pour protéger des matières organiques de l'action de l'oxygène, de la chaleur, de la lumière ou d'un rayonnement riche en énergie.

16. Emploi des composés de formule I selon la revendication 15 comme antioxydants dans des matières polymères organiques ou des huiles minérales ou synthétiques.